# EUROPEAN PATENT APPLICATION

(11) **EP 3 486 323 A1**
(43) Date of publication of application: **22.05.2019**
(21) Application number: 17827554.1
(22) Date of filing: 07.07.2017
(51) Int. Cl.: C12P 7/44, C12N 1/15, C12N 1/19, C12N 1/21, C12P 17/04, C07C 55/28, C07D 307/46, C12N 15/09

(54) **MICROORGANISM HAVING MUCIC ACID-PRODUCING ABILITY, AND METHODS FOR PRODUCING MUCIC ACID, 2,5-FURANDICARBOXYLIC ACID, AND 2,5-FURANDICARBOXYLIC ACID DIESTER**

(30) Priority: 13.07.2016 JP 2016138802
(71) Applicant: Mitsubishi Chemical Corporation, Tokyo 100-8251 (JP)
(72) Inventor: YUNOMURA, Shuichi, Tokyo 100-8251 (JP); TANIGUCHI, Takeshi, Tokyo 100-8251 (JP)
(74) Representative: Hoffmann Eitle
(86) International application number: PCT/JP2017/024963
(87) International publication number: WO 2018/012426

(57) **Abstract**

A method for efficiently producing mucic acid from a common organic raw material such as glucose or galactose is provided. A method for producing mucic acid, including: using glucose or galactose as a starting material or an intermediate; and obtaining mucic acid via UDP-galacturonic acid and galacturonic acid, which are produced sequentially as intermediates. A microorganism modified to have enhanced UDP-galacturonate pyrophosphorylase activity and to have at least one enhanced enzyme activity selected from the group consisting of UDP-glucuronate 4-epimerase activity, UDP-galactose 6-dehydrogenase activity, and galacturonate dehydrogenase activity, as compared to non-modified strains.

SEQUENCE LISTING

<110>  MITSUBISHI CHEMICAL CORPORATION

<120>  Microorganism Having Mucic Acid Producing Ability and Method for
       Producing Mucic Acid, 2,5-Furandicarboxylic Acid, and
       2,5-Furandicarboxylic Acid Diester

<130>  A00095-17282

<150>  JP2016-138802
<151>  2016-07-13

<160>  31

<170>  PatentIn version 3.5

<210>  1
<211>  69
<212>  DNA
<213>  Artificial Sequence

<220>
<223>  gETF primer

<400>  1
gttatgctat ggttatttca taccataagc ctaatggagc gaattatggt gtaggctgga      60

gctgcttcg                                                              69


<210>  2
<211>  70
<212>  DNA
<213>  Artificial Sequence

<220>
<223>  gETR primer

<400>  2
gtttttcttt tcagactcat ttcttacact ccggattcgc gaaaatggat attccgggga      60

tccgtcgacc                                                             70


<210>  3
<211>  60
<212>  DNA
<213>  Artificial Sequence

<220>
<223>  ugdF primer

<400>  3
gaccgaattc attaaagagg agaaaggtac catgaaaatc accatttccg gtactggcta      60


<210>  4
<211>  60
<212>  DNA
<213>  Artificial Sequence

<220>

<223>  ugdR primer

<400>  4
aatggcagcc atagttaatt tctcctggat ccttagtcgc tgccaaagag atcgcgggtg        60


<210>  5
<211>  60
<212>  DNA
<213>  Artificial Sequence

<220>
<223>  galUF primer

<400>  5
gcgactaagg atccaggaga aattaactat ggctgccatt aatacgaaag tcaaaaaagc        60


<210>  6
<211>  66
<212>  DNA
<213>  Artificial Sequence

<220>
<223>  galUR primer

<400>  6
ctgcttttaa attcgtcata gttaatttct cctctgcagt tacttcttaa tgcccatctc        60

ttcttc                                                                    66


<210>  7
<211>  60
<212>  DNA
<213>  Artificial Sequence

<220>
<223>  galFF primer

<400>  7
aagaagtaac tgcagaggag aaattaacta tgacgaattt aaaagcagtt attcctgtag        60


<210>  8
<211>  60
<212>  DNA
<213>  Artificial Sequence

<220>
<223>  galFR primer

<400>  8
ttgtggattg ccatagttaa tttctcctcc atggttattc gcttaacagc ttctcaatac        60


<210>  9
<211>  60
<212>  DNA
<213>  Artificial Sequence

<220>
<223>  pgmF primer

<400>  9
ttaagcgaat aaccatggag gagaaattaa ctatggcaat ccacaatcgt gcaggccaac        60

<210>  10
<211>  60
<212>  DNA
<213>  Artificial Sequence

<220>
<223>  pgmR primer

<400>  10
ctttcgtttt atttgatgcc tctagagcta gcaagctttt acgcgttttt cagaacttcg        60

<210>  11
<211>  28
<212>  DNA
<213>  Artificial Sequence

<220>
<223>  pZAR primer

<400>  11
ggtacctttc tcctctttaa tgaattcg                                          28

<210>  12
<211>  36
<212>  DNA
<213>  Artificial Sequence

<220>
<223>  pZAF primer

<400>  12
gctagctcta gaggcatcaa ataaaacgaa aggctc                                 36

<210>  13
<211>  60
<212>  DNA
<213>  Artificial Sequence

<220>
<223>  agpF primer

<400>  13
ccgttcaact aaggatccag gagaaattaa ctatgcaaac cgtaccggaa ggctatcagc        60

<210>  14
<211>  60
<212>  DNA
<213>  Artificial Sequence

<220>
<223>  agpR primer

<400>  14
agacgggttg gtcatagtta atttctcctg ctagcttatt tcaccgcttc attcaacacg        60

<210>  15
<211>  807
<212>  DNA
<213>  Pseudomonas putida

<400>  15
atgaccacta cccccttcaa tcgcctgctg ctcaccggag ccgcaggcgg cctgggcaag      60

gtccttcgcg aacgcctgaa aggctacgcc gaggtcctgc gcctgtctga catcagcccc     120

atggccccgg ccgcgggccc gcatgaagaa gtcattacct gtgacctggc cgacaaggct     180

gcggtgcata ccctggtcga gggcgtagac gccatcatcc actttggcgg ggtttctacc     240

gaacacgcct tcgaagagat tctcggcccc aatatctgcg gcgtgttcca cgtgtacgag     300

gcggcgcgca agcacggggt caagcgcatc atcttcgcca gctccaacca caccatcggt     360

ttctatcgcc aggatgagcg catcgacgct cacgcgccgc gccggcccga cagctattac     420

gggctgtcca agtgctacgg cgaagatgtg gccagcttct actttgaccg ctacggcatc     480

gagaccgtca gcattcgcat cggctcgtcg ttcccgcagc cacagaacct gcgcatgctc     540

tgcacctggc tcagttacga cgacctggtg cagttgatcg aacgcgggct gttcaccccc     600

ggggttggcc acaccatcgt ctacggcgcc tccgacaatc gcaccgtgtg gtgggacaac     660

cgccatgccg cgcacctggg ctatgtaccc aaggacagct ccgaaacctt ccgcgcagcc     720

gtggaggccc aaccggcacc cgccgccgat gacccgagca tggtctacca gggcggcgct     780

ttcgccgtgg ccggcccgtt caactaa                                         807


<210>  16
<211>  1893
<212>  DNA
<213>  Leishmania major

<400>  16
atgaccaacc cgtctaactc taacctgcaa gctctgcgtg aagaactgtg caccccgggt      60

ctggaccagg gtcacctgtt cgaaggttgg ccggaaaccg ttgacgaatg caacgaacgt     120

cagatcgctc tgctgaccga cctgtacatg ttctctaaca tgtacccggg tggtgttgct     180

cagtacatcc gtaacggtca cgaactgctg gctcgtgaat ctgaagaagt tgacttcgct     240

gctctggaaa tgccgccgct gatcttcgaa gctccgtctc tgcaccgtcg taccgctgaa     300

cgtaccgctc tggaaaacgc tggtactgct atgctgtgca aaaccgtttt cgttctggtt     360

gctggtggtc tgggtgaacg tctgggttac tcttctatca aagtttctct gccggttgaa     420

accgctacca acaccaccta cctggcttac tacctgcgtt gggctcagcg tgttggtggt     480

aaagaagttc cgttcgttat catgacctct gacgacaccc acgaccgtac cctgcaactg     540

ctgcgtgaac tgcaactgga agttccgaac ctgcacgttc tgaaacaggg tcaggttttc     600

tgcttcgctg actctgctgc tcacctggct ctggacgaaa ccggtaaact gctgcgtaaa 660

ccgcacggtc acggtgacgt tcactctctg atctacaacg ctaccgttaa acgtgacgtt 720

gttccggact ctggtgacgg tactgctacc gctcagccgc tggttaacga ctggctggct 780

gctggttacg aatctatcgt tttcatccag gacaccaacg ctggtgctac catcaccatc 840

ccgatctctc tggctctgtc tgctgaacac tctctggaca tgaacttcac ctgcatcccg 900

cgtgttccga aagaaccgat cggtctgctg tgccgtacca aaaaaaactc tggtgacccg 960

tggctggttg ctaacgttga atacaacgtt ttcgctgaag tttctcgtgc tctgaacaaa 1020

gacggtggtg acgaagtttc tgacccgacc ggtttctctc cgttcccggg ttctgttaac 1080

accctggttt tcaaactgtc ttcttacgtt gaccgtctgc gtgaatctca cggtatcgtt 1140

ccggaattta tcaacccgaa atactctgac gaaacccgtc gttctttcaa aaaaccggct 1200

cgtatcgaat ctctgatgca ggacatcgct ctgctgttct ctgaagacga ctaccgtgtt 1260

ggtggtactg ttttcgaacg tttctcttac cagccggtta aaaactctct ggaagaagct 1320

gctggtctgg ttgctcaggg taacggtgct tactgcgctg ctaccggtga agctgctttc 1380

tacgaactgc aacgtcgtcg tctgaaagct atcggtctgc cgctgttcta ctcttctcag 1440

ccggaagtta ccgttgctaa agacgctttc ggtgttcgtc tgttcccgat catcgttctg 1500

gacaccgttt gcgcttcttc tggttctctg gacgacctgg ctcgtgtttt cccgaccccg 1560

gaaaaagttc acatcgacca gcactctacc ctgatcgttg aaggtcgtgt tatcatcgaa 1620

tctctggaac tgtacggtgc tctgaccatc cgtggtccga ccgactctat ggctctgccg 1680

cacgttgttc gtaacgctgt tgttcgtaac gctggttggt ctgttcacgc tatcctgtct 1740

ctgtgcgctg gtcgtgactc tcgtctgtct gaagttgacc gtatccgtgg tttcgttctg 1800

aaaaaaaccg ctatggctgt tatggactgc aacaccaaag gtgaatctga agctggtgct 1860

ccgtctggtg ctgctgaccc ggctaaactg taa 1893

<210> 17
<211> 1071
<212> DNA
<213> Streptococcus pneumoniae

<400> 17
atgcgtggta aaaccatcct gatcaccggt gttgctggtt tcatcggttc taacctggtt 60

cgtaaactgc tggaactgga aggtactatg accatcatcg gtctggacaa cctgaacgac 120

tactacgaca tcgctctgaa agactaccgt ctgctgcaac tgaacaaact gatccaggac 180

tacccggaaa aaaactggat cttcatcaaa ggtgacatcg ctgacaaaat gctgatcaac 240

aaaatcttca aatcttacca cccgcagatc gttgttaacc tggctgctca ggctggtgtt 300

cgttactcta tcaccaaccc ggacgtttac atcgaatcta acatcgttgg tttccacaac 360

gttctggaag cgtgccgtca ctcttacgaa atctacgacg gtggtgttga acacctggtt 420

tacgcttctt cttcttctgt ttacggtggt aacaaaaaaa tcccgttcgc taccgaagac 480

aaagttgaca acccgatctc tctgtacgct gctaccaaaa aaaccaacga actgcaagcg 540

tacgcttact ctaaaatcta caacatcccg tctaccggtc tgcgtttctt caccgtttac 600

ggtccggctg gtcgtccgga catggcttac ttcggtttca ccgacaaact gcgttctggt 660

tctgacatcg aaatctacaa ccacggtaac tgcaaacgtg acttcaccta catcgacgac 720

atcgttgaag gtatcgttcg tgttatgcag tctgctccga aaaaactggt tggttctgac 780

aacctgccgc tggctccgta caaagtttac aacatcggta actctaaacc ggaaaacctg 840

ctggacttcg ttgacgttct gcaacaggaa ctgatcaaag ctggtgttct gccggaaaac 900

tacgacttcg actctcacaa aaaactggtt ccgatgcagc cgggtgacgt tccggttacc 960

tacgctgaca cctctgacct ggaacgtgac ttcggtttca aaccgaaaac ctctctgcgt 1020

gaaggtctgc gtaaattcgc tgaatggtac aaagactact acaacgttta a 1071

<210> 18
<211> 59
<212> DNA
<213> Artificial Sequence

<220>
<223> udhF primer

<400> 18
cgaattcatt aaagaggaga aaggtaccat gaccactacc cccttcaatc gcctgctgc 59

<210> 19
<211> 60
<212> DNA
<213> Artificial Sequence

<220>
<223> udhR primer

<400> 19
gtacggtttg catagttaat ttctcctgga tccttagttg aacgggccgg ccacggcgaa 60

<210> 20
<211> 60
<212> DNA
<213> Artificial Sequence

<220>
<223> LmjF17.1160F primer

<400> 20
gaagcggtga aataagctag caggagaaat taactatgac caacccgtct aactctaacc 60

<210> 21
<211> 60

<212>  DNA
<213>  Artificial Sequence

<220>
<223>  LmjF17.1160R primer

<400>  21
ggttttacca cgcatagtta atttctcctc catggttaca gtttagccgg gtcagcagca        60


<210>  22
<211>  60
<212>  DNA
<213>  Artificial Sequence

<220>
<223>  cap1JF primer

<400>  22
cggctaaact gtaaccatgg aggagaaatt aactatgcgt ggtaaaacca tcctgatcac        60


<210>  23
<211>  56
<212>  DNA
<213>  Artificial Sequence

<220>
<223>  cap1JR primer

<400>  23
gagcctttcg ttttatttga tgcctctaga ttaaacgttg tagtagtctt tgtacc        56


<210>  24
<211>  60
<212>  DNA
<213>  Artificial Sequence

<220>
<223>  agpR94HR primer

<400>  24
gtaataaaga actgtgcggt cgcaacggta tgttgcagac tattggcata ggcataaacg        60


<210>  25
<211>  60
<212>  DNA
<213>  Artificial Sequence

<220>
<223>  agpR94HF primer

<400>  25
cgtttatgcc tatgccaata gtctgcaaca taccgttgcg accgcacagt tctttattac        60


<210>  26
<211>  54
<212>  DNA
<213>  Artificial Sequence

<220>
<223>  agpM123VR primer

<400>  26
gatcaccggg ttaaaggttg ggtccacagt gcccattttt tcctggtgat gcac          54


<210>  27
<211>  54
<212>  DNA
<213>  Artificial Sequence

<220>
<223>  agpM123VF primer

<400>  27
gtgcatcacc aggaaaaaat gggcactgtg gacccaacct ttaacccggt gatc          54


<210>  28
<211>  54
<212>  DNA
<213>  Artificial Sequence

<220>
<223>  agpM123AR primer

<400>  28
gatcaccggg ttaaaggttg ggtccgcagt gcccattttt tcctggtgat gcac          54


<210>  29
<211>  54
<212>  DNA
<213>  Artificial Sequence

<220>
<223>  agpM123AF primer

<400>  29
gtgcatcacc aggaaaaaat gggcactgcg gacccaacct ttaacccggt gatc          54


<210>  30
<211>  53
<212>  DNA
<213>  Artificial Sequence

<220>
<223>  agpE196DR primer

<400>  30
gactttcagc ggcccggaaa cacctggatc ttgttgatac ttggcgctaa agg           53


<210>  31
<211>  53
<212>  DNA
<213>  Artificial Sequence

<220>
<223>  agpE196DF primer

<400>  31
cctttagcgc caagtatcaa caagatccag gtgtttccgg gccgctgaaa gtc                53

## Description

### TECHNICAL FIELD

The present invention relates to a novel microorganism having mucic acid-producing ability, and a method for producing mucic acid using it. The present invention also relates to a method for producing 2,5-furandicarboxylic acid or 2,5-furandicarboxylic acid diester using mucic acid obtained by this production method as a raw material.

### BACKGROUND ART

Mucic acid is the dicarboxylic acid wherein the formyl group (-CHO) at the 1-position and the hydroxymethyl group (-CH₂OH) at the end of the backbone in galactose are both converted to carboxyl groups, and also called (meso)-galactaric acid. Mucic acid is expected to be a biomass-derived key compound which can be induced to a variety of useful chemicals, for example, as a raw material of 2,5-furandicarboxylic acid and 2,5-furandicarboxylic acid diester, which are drawing attention as novel polymer raw materials.

It is known that mucic acid can be synthesized by nitric acid oxidation of galactose. However, this method has problems such as the fact that the oxidation reaction is carried out under high temperature and high pressure conditions, and the fact that nitrogen oxides, which cause environmental pollution, are produced as byproducts. Thus, production by enzymatic conversion or fermentation has been attempted to enable highly selective synthesis at normal temperature and pressure.

Patent Document 1 discloses a method in which mucic acid is produced using galacturonic acid as a raw material by use of a fungus in which the galacturonate dehydrogenase gene is introduced.

Non-patent Document 1 describes that, by introducing the UDP-galacturonate pyrophosphorylase gene into *E. coli* and allowing its expression, an activity that produces galacturonate-1-phosphate using UDP-galacturonic acid as a substrate could be detected.

Non-patent Document 2 describes that, by introducing the UDP-glucuronate 4-epimerase gene into *E. coli* and allowing its expression, an activity that produces UDP-galacturonic acid using UDP-glucuronic acid as a substrate could be detected.

Non-patent Document 3 describes that UDP-glucose 6-dehydrogenase derived from the archaebacterium *Haloferax volcanii* recognized not only UDP-glucose, but also UDP-galactose as a substrate, leading to detection of the UDP-galactose 6-dehydrogenase activity.

### PRIOR ART DOCUMENTS

### [Patent Document]

Patent Document 1: WO 2010/072902

### [Non-patent Documents]

Non-patent Document 1: J Biol Chem., 2010, Vol. 285(2), p 878-87
Non-patent Document 2: Mol Microbiol., 1999, Vol. 31(2), p 703-13
Non-patent Document 3: Mol Microbiol., 2010, Vol. 75(4), p 1047-58

### SUMMARY OF THE INVENTION

### PROBLEMS TO BE SOLVED BY THE INVENTION

However, the method described in Patent Document 1 requires use of galacturonic acid as a raw material, so that mucic acid cannot be produced from a common organic raw material. Thus, a method for producing mucic acid using an inexpensive, easily available organic raw material such as glucose has been demanded.

Regarding the method described in Non-patent Document 1, expression of the UDP-galacturonate pyrophosphorylase gene and confirmation of its enzyme activity have been reported, but there is neither description nor suggestion on the production of mucic acid.

Regarding the method described in Non-patent Document 2, expression of the UDP-glucuronate 4-epimerase gene and confirmation of its enzyme activity have been reported, but there is neither description nor suggestion on the production of mucic acid.

Regarding the method described in Non-patent Document 3, UDP-glucose 6-dehydrogenase having UDP-galactose 6-dehydrogenase activity has been disclosed, but there is neither description nor suggestion on the production of mucic acid.

An object of the present invention is to provide a method for efficiently producing mucic acid using a common organic raw material such as glucose or galactose.

### MEANS FOR SOLVING THE PROBLEMS

As a result of intensive study to solve the above problem, the present inventors discovered that mucic acid can be produced by a novel reaction process comprising: using glucose or galactose as a starting material; and obtaining mucic acid via UDP-galacturonic acid and galacturonic acid, which are produced sequentially as intermediates.

The present inventors also discovered that, by modifying a microorganism such that it has enhanced UDP-galacturonate pyrophosphorylase activity and such that it also has at least one enhanced enzyme activity selected from the group consisting of UDP-glucuronate 4-epimerase activity, UDP-galactose 6-dehydrogenase activity, and galacturonate dehydrogenase activity, a metabolic pathway for biosynthesis of mucic acid from glucose or galactose can be constructed, which is preferred for a production method for mucic acid comprising the above reaction process, thereby completing the present invention.

That is, according to the present invention, the following inventions are provided.
[1] A method for producing mucic acid, comprising:
   using glucose or galactose as a starting material or an intermediate; and
   obtaining mucic acid via UDP-galacturonic acid and galacturonic acid, which are produced sequentially as intermediates.
[2] The method for producing mucic acid according to [1], wherein
   glucose is used as a starting material or an intermediate, and
   mucic acid is obtained via UDP-glucose, UDP-galacturonic acid, and galacturonic acid, which are produced sequentially as intermediates.
[3] The method for producing mucic acid according to [2], wherein mucic acid is obtained via UDP-glucuronic acid, which is produced as an intermediate after UDP-glucose and before UDP-galacturonic acid.
[4] The method for producing mucic acid according to [2], wherein mucic acid is obtained via UDP-galactose, which is produced as an intermediate after UDP-glucose and before UDP-galacturonic acid.
[5] The method for producing mucic acid according to [1], wherein galactose is used as a starting material or an intermediate, and mucic acid is obtained via UDP-galactose, UDP-galacturonic acid, and galacturonic acid, which are produced sequentially as intermediates.
[6] A microorganism modified to have enhanced UDP-galacturonate pyrophosphorylase activity and to have at least one enhanced enzyme activity selected from the group consisting of UDP-glucuronate 4-epimerase activity, UDP-galactose 6-dehydrogenase activity, and galacturonate dehydrogenase activity, as compared to non-modified strains.
[7] The microorganism according to [6], wherein
   the microorganism is modified to have enhanced UDP-glucuronate 4-epimerase activity, UDP-galacturonate pyrophosphorylase activity, and galacturonate dehydrogenase activity, as compared to non-modified strains.
[8] The microorganism according to [7], wherein
   the microorganism is modified to further have at least one enhanced enzyme activity selected from the group consisting of glucokinase activity, phosphoglucomutase activity, glucose-1-phosphate uridylyltransferase activity, UDP-glucose 6-dehydrogenase activity, and galacturonate-1-phosphate phosphatase activity, as compared to non-modified strains.
[9] The microorganism according to [7] or [8], wherein
   the microorganism is modified to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, hexose-1-phosphate uridylyltransferase activity, UDP-glucose 4-epimerase activity, UDP-glucuronic acid dehydrogenase activity, galacturonate isomerase activity, galacturonate reductase activity, and galactarate dehydratase activity, as compared to non-modified strains.
[10] The microorganism according to [6], wherein
   the microorganism is modified to have enhanced UDP-galactose 6-dehydrogenase activity, UDP-galacturonate pyrophosphorylase activity, and galacturonate dehydrogenase activity, as compared to non-modified strains.
[11] The microorganism according to [10], wherein
   the microorganism is modified to further have at least one enhanced enzyme activity selected from the group consisting of glucokinase activity, phosphoglucomutase activity, glucose-1-phosphate uridylyltransferase activity, hexose-1-phosphate uridylyltransferase activity, UDP-glucose 4-epimerase activity, galactokinase activity, UDP-galactose pyrophosphorylase activity, and galacturonate-1-phosphate phosphatase activity, as compared to non-modified strains.
[12] The microorganism according to [10] or [11], wherein
   the microorganism is modified to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, UDP-glucose 6-dehydrogenase activity, UDP-glucuronic acid dehydrogenase activity, galacturonate isomerase activity, galacturonate reductase activity, and galactarate dehydratase activity, as compared to non-modified strains.
[13] The microorganism according to any one of [6] to [12], wherein
   the microorganism is at least one selected from the group consisting of coliform bacteria, coryneform bacteria, bacteria belonging to the genus *Bacillus,* bacteria belonging to the genus *Lactobacillus,* bacteria belonging to the genus *Actinobacillus,* bacteria belonging to the genus *Pseudomonas,* filamentous fungi, and yeasts.
[14] A method for producing mucic acid, comprising:
   allowing the microorganism according to any one of [6] to [13] or a treated product of the microorganism to act on an organic raw material in an aqueous medium.
[15] The method for producing mucic acid according to [14], wherein
   the organic raw material contains at least one selected from the group consisting of glucose, sucrose, galactose, and lactose.
[16] A method for producing 2,5-furandicarboxylic acid, comprising the steps of:
   (i) producing mucic acid by the method according to any one of [1] to [5], [14], and [15]; and
   (ii) converting the mucic acid obtained in the step (i) into 2,5-furandicarboxylic acid.
[17] A method for producing 2,5-furandicarboxylic acid diester, comprising the steps of:
   (i) producing mucic acid by the method according to any one of [1] to [5], [14], and [15]; and
   (ii) converting the mucic acid obtained in the step (i) into 2,5-furandicarboxylic acid diester.

### EFFECT OF THE INVENTION

By the present invention, mucic acid can be produced from a common organic raw material such as glucose or galactose. Further, a microorganism capable of biosynthesis of mucic acid from a common organic raw material such as glucose or galactose can be prepared, and, by using such a microorganism of the present invention, mucic acid can be efficiently produced.

### BRIEF DESCRIPTION OF THE DRAWING

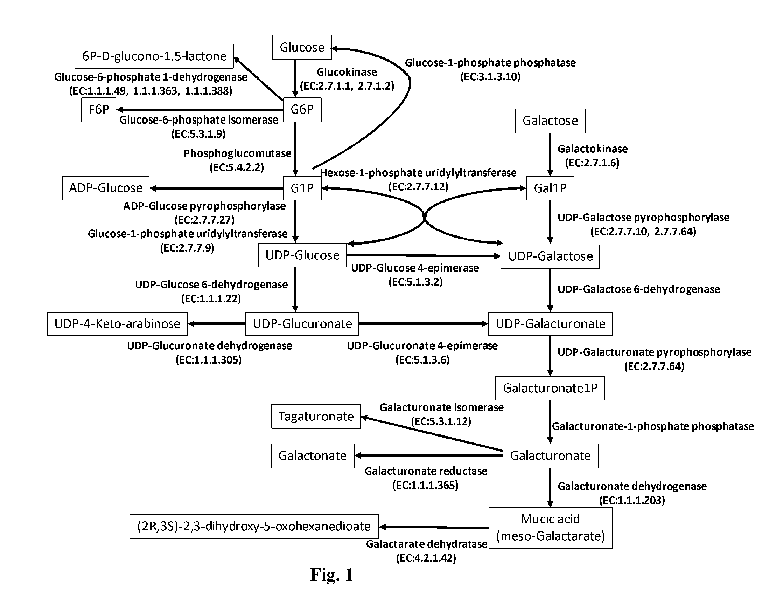

Fig. 1 is a diagram showing the pathways related to the synthesis of mucic acid and production of its byproducts in the production method of the present invention.

### MODE FOR CARRYING OUT THE INVENTION

Embodiments of the present invention are described below in detail.

### <First Method for Producing Mucic Acid>

The method for producing mucic acid discovered by the present inventors includes a reaction process comprising: using glucose or galactose as a starting material or an intermediate; and obtaining mucic acid via UDP-galacturonic acid and galacturonic acid, which are produced sequentially as intermediates. By such a reaction process, production of mucic acid using a common organic raw material such as glucose or galactose is possible.

In cases where glucose is used as a starting material or an intermediate, the method preferably comprises a reaction process in which mucic acid is obtained via UDP-glucose, UDP-galacturonic acid, and galacturonic acid, which are produced sequentially as intermediates. In such cases, the method more preferably comprises a reaction process in which mucic acid is obtained via UDP-glucuronic acid, which is produced as an intermediate after UDP-glucose and before UDP-galacturonic acid, or a reaction process in which mucic acid is obtained via UDP-galactose, which is produced as an intermediate after UDP-glucose and before UDP-galacturonic acid.

A reaction pathway in a case where glucose is used as a starting material or an intermediate is described with reference to Fig. 1, but the reaction pathway is not limited thereto.

The reaction may be started using glucose as a starting material, or may be started from glucose produced by an appropriate reaction from another starting material. Glucose undergoes, for example, phosphorylation reaction to become glucose-6-phosphate, and then undergoes phosphotransfer reaction to become glucose-1-phosphate, followed by undergoing dephosphorylation and UDP addition reaction to become UDP-glucose. The UDP-glucose undergoes, for example, oxidation reaction to become UDP-glucuronic acid, and then undergoes isomerization reaction to become UDP-galacturonic acid. Alternatively, the UDP-glucose first undergoes, for example, isomerization reaction to become UDP-galactose, and then undergoes oxidation reaction to become UDP-galacturonic acid. The UDP-galacturonic acid undergoes, for example, de-UDP and phosphate addition reaction to become galacturonate-1-phosphate, and then undergoes dephosphorylation reaction to become galacturonic acid. By allowing the galacturonic acid to undergo oxidation reaction, mucic acid is produced as the substance of interest.

In cases where galactose is used as a starting material or an intermediate, the method preferably comprises a reaction process in which mucic acid is obtained via UDP-galactose, UDP-galacturonic acid, and galacturonic acid, which are produced sequentially as intermediates.

A reaction pathway in a case where galactose is used as a starting material or an intermediate is described with reference to Fig. 1, but the reaction pathway is not limited thereto.

The reaction may be started using galactose as a starting material, or may be started from galactose produced by an appropriate reaction from another starting material. The galactose undergoes, for example, phosphorylation reaction to become galactose-1-phosphate, and then undergoes dephosphorylation and UDP addition reaction to become UDP-galactose, followed by undergoing oxidation reaction to become UDP-galacturonic acid. As described above, the UDP-galacturonic acid undergoes, for example, de-UDP and phosphate addition reaction to become galacturonate-1-phosphate, and then undergoes dephosphorylation reaction to become galacturonic acid. By allowing the galacturonic acid to undergo oxidation reaction, mucic acid is produced as the substance of interest.

The first method for producing mucic acid of the present invention may be carried out any method, and examples of the method include a method in which biosynthesis is carried out utilizing a metabolic pathway of a microorganism, an in vitro method using an enzyme reaction, and a method in which chemical synthesis is carried out. Among these, biosynthesis by a microorganism is especially preferred. It is described below in detail.

### <Microorganism of Present Invention>

The microorganism of the present invention is a microorganism modified to have enhanced UDP-galacturonate pyrophosphorylase activity and to have at least one enhanced enzyme activity selected from the group consisting of UDP-glucuronate 4-epimerase activity, UDP-galactose 6-dehydrogenase activity, and galacturonate dehydrogenase activity, as compared to non-modified strains.

Biosynthetic pathways for UDP-glucose have been found in a variety of microorganisms so far, and it is generally known that UDP-glucuronic acid and UDP-galactose can be synthesized from this metabolite.

In view of this, the present inventors discovered that, by modifying a microorganism such that it has enhanced UDP-galacturonate pyrophosphorylase activity and such that it also has at least one enhanced enzyme activity selected from the group consisting of UDP-glucuronate 4-epimerase activity, UDP-galactose 6-dehydrogenase activity, and galacturonate dehydrogenase activity, a metabolic pathway for synthesis of mucic acid from UDP-glucuronic acid or UDP-galactose can be constructed. Here, it is preferred to modify the microorganism such that it has enhanced UDP-glucuronate 4-epimerase activity, UDP-galacturonate pyrophosphorylase activity, and galacturonate dehydrogenase activity, and, in this case, a metabolic pathway for synthesis of mucic acid from UDP-glucuronic acid is constructed. It is also preferred to modify the microorganism such that it has enhanced UDP-galactose 6-dehydrogenase activity, UDP-galacturonate pyrophosphorylase activity, and galacturonate dehydrogenase activity, and, in this case, a metabolic pathway for synthesis of mucic acid from UDP-galactose is constructed.

For the synthesis of mucic acid with these metabolic pathways, the galacturonate-1-phosphate produced by the UDP-galacturonate pyrophosphorylase needs to be converted to galacturonic acid by dephosphorylation. In general, a phosphatase that catalyzes dephosphorylation reaction has low substrate specificity. Therefore, usually, this enzyme retained by the host microorganism recognizes the galacturonate-1-phosphate as a substrate, and has galacturonate-1-phosphate phosphatase activity.

As described above, by the modification for enhancement of the enzyme activities, a microorganism having mucic acid-producing ability can be prepared. In the present invention, the "mucic acid-producing ability" means that, when a microorganism is cultured in a medium, the microorganism can produce and accumulate mucic acid in the medium.

Mucic acid is the dicarboxylic acid wherein the formyl group (-CHO) at the 1-position and the hydroxymethyl group (-CH₂OH) at the end of the backbone in galactose are both converted to carboxyl groups, and also called (meso)-galactaric acid.

Examples of means for the enhancement of the enzyme activities include mutation treatment and genetic recombination treatment. The enhancement can be carried out by employing a known method such as introduction of a foreign gene encoding each enzyme and/or enhancement of expression of an endogenous gene(s).

The microorganism used in the present invention is not limited, and preferably a microorganism having a metabolic pathway capable of synthesis of UDP-glucuronic acid or UDP-galactose. More specifically, the microorganism is preferably a microorganism selected from the group consisting of coliform bacteria, coryneform bacteria, bacteria belonging to the genus *Bacillus,* bacteria belonging to the genus *Lactobacillus,* bacteria belonging to the genus *Actinobacillus,* bacteria belonging to the genus *Pseudomonas,* filamentous fungi, and yeasts.

Among these, the microorganism is preferably at least one selected from the group consisting of coliform bacteria, coryneform bacteria, bacteria belonging to the genus *Bacillus,* filamentous fungi, and yeasts, more preferably coliform bacteria, coryneform bacteria, and yeasts, especially preferably coliform bacteria and yeasts.

Examples of the coliform bacteria that can be used in the present invention include *Escherichia coli* such as the K12 strain, B strain, and their derivatives BW25113, HB101, DH5α, JM109, JM110, BL21(DE3), TH2, and TOP10.

The coryneform bacteria that can be used in the present invention are not limited as long as they are classified as coryneform bacteria. Examples of the coryneform bacteria include bacteria belonging to the genus *Corynebacterium,* bacteria belonging to the genus *Brevibacterium,* and bacteria belonging to the genus *Arthrobacter.* Among these, those belonging to the genus *Corynebacterium* or *Brevibacterium* are preferred. Among these, bacteria classified as *Corynebacterium glutamicum, Brevibacterium flavum, Brevibacterium ammoniagenes,* or *Brevibacterium lactofermentum* are more preferred.

Especially preferred specific examples of the coryneform bacteria that can be used in the present invention include *Brevibacterium flavum* MJ-233 (FERM BP-1497) and MJ-233 AB-41 (FERM BP-1498); *Brevibacterium ammoniagenes* ATCC6872; *Corynebacterium glutamicum* ATCC31831; and *Brevibacterium lactofermentum* ATCC13869. At present, *Brevibacterium flavum* is classified as *Corynebacterium glutamicum* in some cases (Int J Syst Bacteriol., 1991, Vol.41, p 255-260). Therefore, in the present invention, the *Brevibacterium flavum* MJ-233 strain and its mutant strain MJ-233 AB-41 are regarded as the same strains as the *Corynebacterium glutamicum* MJ-233 strain and MJ-233 AB-41 strain, respectively.

The *Brevibacterium flavum* MJ-233 strain was deposited with Fermentation Research Institute, Agency of Industrial Science and Technology, Ministry of International Trade and Industry (currently International Patent Organism Depositary, National Institute of Technology and Evaluation) (Room #120, 2-5-8 Kazusakamatari, Kisarazu-shi, Chiba 292-0818, Japan) as of April 28, 1975 under the accession No. FERM P-3068, and the deposition was converted to international deposition under the Budapest Treaty as of May 1, 1981 under the accession No. FERM BP-1497.

Examples of the bacteria belonging to the genus *Bacillus* that can be used in the present invention include *Bacillus subtilis, Bacillus licheniformis,* and *Bacillus amyloliquefaciens.*

Examples of the bacteria belonging to the genus *Lactobacillus* that can be used in the present invention include *Lactobacillus helveticus, Lactobacillus acidophilus,* and *Lactobacillus casei.*

Examples of the bacteria belonging to the genus *Actinobacillus* that can be used in the present invention include *Actinobacillus succinogenes.*

Examples of the bacteria belonging to the genus *Pseudomonas* that can be used in the present invention include *Pseudomonas putida, Pseudomonas veronii, Pseudomonas viridiflava,* and *Pseudomonas fluorescens.*

Examples of the filamentous fungi that can be used in the present invention include the genus *Aspergillus,* the genus *Penicillium,* the genus *Rhizopus,* and the genus *Hypocrea.*

Examples of the genus *Aspergillus* include *Aspergillus niger* and *Aspergillus oryzae.*

Examples of the genus *Penicillium* include *Penicillium chrysogenum* and *Penicillium simplicissimum.*

Examples of the genus *Rhizopus* include *Rhizopus oryzae.*

Examples of the genus *Hypocrea* include *Hypocrea jecorina.* The imperfect state of this fungus is *Trichoderma reesei.*

Examples of the yeasts that can be used in the present invention include the genus *Saccharomyces,* the genus *Schizosaccharomyces,* the genus *Candida,* the genus *Pichia,* the genus *Kluyveromyces,* the genus *Yarrowia,* and the genus *Zygosaccharomyces.*

Examples of the genus *Saccharomyces* include *Saccharomyces cerevisiae, Saccharomyces uvarum,* and *Saccharomyces bayanus.*

Examples of the genus *Schizosaccharomyces* include *Schizosaccharomyces pombe.*

Examples of the genus *Candida* include *Candida albicans, Candida utilis, Candida boidinii, Candida sonorensis,* and *Candida glabrata.*

Examples of the genus *Pichia* include *Pichia pastoris* and *Pichia stipitis.*

Examples of the genus *Kluyveromyces* include *Kluyveromyces lactis, Kluyveromyces marxianus,* and *Kluyveromyces thermotolerans.*

Examples of the genus *Yarrowia* include *Yarrowia lipolytica.*

Examples of the genus *Zygosaccharomyces* include *Zygosaccharomyces bailii* and *Zygosaccharomyces rouxii.*

The microorganism is not limited to a wild strain, and may be any strain including mutant strains obtained by ordinary mutation treatment such as UV irradiation or NTG treatment; and recombinant strains induced by a genetic method such as cell fusion or genetic recombination.

As described above, the microorganism of the present invention can be obtained by modifying a microorganism such that it has enhanced UDP-glucuronate 4-epimerase activity, UDP-galacturonate pyrophosphorylase activity, and galacturonate dehydrogenase activity, as compared to non-modified strains, or such that it has enhanced UDP-galactose 6-dehydrogenase activity, UDP-galacturonate pyrophosphorylase activity, and galacturonate dehydrogenase activity, as compared to non-modified strains.

In the present invention, the "UDP-glucuronate 4-epimerase (hereinafter also referred to as UDP-GLE) activity" means the activity that catalyzes a reaction to isomerize UDP-glucuronic acid to produce UDP-galacturonic acid (EC:5.1.3.6). The "enhanced UDP-GLE activity" means that the UDP-GLE activity is increased compared to non-modified strains. The UDP-GLE activity is preferably increased not less than 1.5-fold, more preferably increased not less than 3-fold, per unit microbial weight compared to non-modified strains. The fact that the UDP-GLE activity is given or enhanced can be confirmed by measuring the UDP-GLE activity by a known method such as the method by Munoz et al. (Mol. Microbiol., 1999, Vol. 31(2), p 703-13).

Examples of the method for enhancing the UDP-GLE activity include a method in which the parental strain is treated with a mutagen, a method in which the copy number of the *caplJ* gene, which encodes UDP-GLE, is increased, and a method in which the *caplJ* gene or the promoter of the *caplJ* gene is modified. A plurality of these methods for enhancing the UDP-GLE activity may be used in combination.

A specific method for preparing a strain modified to have enhanced UDP-GLE activity is described below. The strain having enhanced UDP-GLE activity can be obtained by treating the parent strain with a mutagen ordinarily used for mutation treatment, such as N-methyl-N'-nitro-N-nitrosoguanidine (NTG) or ethyl methanesulfonate (EMS), and selecting a strain having increased UDP-GLE activity.

The strain having enhanced UDP-GLE activity can also be obtained by modification using the *caplJ* gene. More specifically, it can be achieved by increasing the copy number of the *caplJ* gene. The copy number can be increased by transformation with a vector containing the *caplJ* gene, or by introduction of the gene into the chromosome by a method such as homologous recombination, and increasing its copy number on the chromosome.

Preparation of the strain having enhanced UDP-GLE activity can also be achieved by introducing a mutation into the *caplJ* gene on the chromosome or on a plasmid vector to increase the UDP-GLE activity per protein molecule encoded by the *caplJ* gene.

Preparation of the strain having enhanced expression of the *caplJ* gene can also be achieved, for example, by introducing a mutation(s) to the promoter of the *caplJ* gene on the chromosome or on a plasmid vector, or by replacing the promoter with a stronger promoter, to increase expression of the *caplJ* gene.

The *caplJ* gene to be used for the enhancement of the UDP-GLE activity is not limited as long as it encodes a protein having UDP-GLE activity, and examples of the gene include the gene derived from *Streptococcus pneumoniae.*

A *caplJ* gene derived from a bacterium other than *Streptococcus pneumoniae,* or from another microorganism, animal, or plant, may also be used. The *caplJ* gene derived from a microorganism, animal, or plant may be, for example, a gene whose base sequence has already been determined, or a gene encoding a protein having UDP-GLE activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the UDP-GLE activity, a plurality of types of *caplJ* genes derived from a different microorganism(s), animal(s), and/or plant(s) may be used.

By inserting the thus isolated gene encoding UDP-GLE into a known expression vector in a manner allowing its expression, a UDP-GLE expression vector can be provided. By performing transformation with this expression vector, a strain modified such that the UDP-GLE activity is enhanced can be obtained. Alternatively, by incorporating DNA encoding UDP-GLE into chromosomal DNA of a host microorganism by homologous recombination or the like in a manner allowing its expression, a strain modified to have enhanced UDP-GLE activity can be obtained. The transformation and the homologous recombination can be carried out according to ordinary methods known to those skilled in the art.

In cases where the *caplJ* gene is introduced into the chromosome or plasmid, an appropriate promoter is incorporated into the 5'-side upstream of the gene, and, more preferably, a terminator is incorporated into the 3'-side downstream of the gene. The promoter and the terminator are not limited as long as they are a promoter and a terminator known to function in the microorganism used as the host. They may be the promoter and the terminator of the *caplJ* gene itself, or may be substituted by another promoter and another terminator. Vectors, promoters, terminators, and the like that can be used in these microorganisms are described in detail in, for example, "Fundamental Microbiology 8: Genetic Engineering. Kyoritsu Shuppan Co., Ltd."

In the present invention, the "UDP-galacturonate pyrophosphorylase (hereinafter also referred to as UDP-GalAPPase) activity" means the activity that catalyzes a reaction to produce galacturonate-1-phosphate from UDP-glucuronic acid (EC:2.7.7.64). The "enhanced UDP-GalAPPase activity" means that the UDP-GalAPPase activity is increased compared to non-modified strains. The UDP-GalAPPase activity is preferably increased not less than 1.5-fold, more preferably increased not less than 3-fold, per unit microbial weight compared to non-modified strains. The fact that the UDP-GalAPPase activity is enhanced can be confirmed by measuring the UDP-GalAPPase activity by a known method such as the method by Damerow et al. (J. Biol. Chem., 2010, Vol. 285(2), p 878-87).

The strain modified such that the UDP-GalAPPase activity is enhanced can be prepared by the same method as the above-described method for enhancing the UDP-GLE activity.

The gene to be used for the enhancement of the UDP-GalAPPase activity is not limited as long as it encodes a protein having UDP-GalAPPase activity, and examples of the gene include the *LmjF17.1160* gene derived from *Leishmania major.*

A gene derived from a bacterium other than *Leishmania major,* or from another microorganism, animal, or plant, may also be used. The gene may be, for example, a gene encoding a protein having UDP-GalAPPase activity, which gene is isolated from the chromosome of a microorganism, animal, plant, or the like based on homology to the *LmjF17.1160* gene or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the UDP-GalAPPase activity, a plurality of types of genes derived from a different microorganism(s), animal(s), and/or plant(s) may be used.

In the present invention, the "galacturonate dehydrogenase (hereinafter also referred to as GalADH) activity" means the activity that catalyzes a reaction to oxidize galacturonic acid to produce mucic acid (EC:1.1.1.203). The "enhanced GalADH activity" means that the GalADH activity is increased compared to non-modified strains. The GalADH activity is preferably increased not less than 1.5-fold, more preferably increased not less than 3-fold, per unit microbial weight compared to non-modified strains. The fact that the GalADH activity is enhanced can be confirmed by measuring the GalADH activity by a known method such as the method by Yoon et al. (J. Acteriol., 2009, Vol. 191(5), p 1565-73) .

The strain modified such that the GalADH activity is enhanced can be prepared by the same method as the above-described method for enhancing the UDP-GLE activity.

The gene to be used for the enhancement of the GalADH activity is not limited as long as it encodes a protein having GalADH activity, and examples of the gene include the *udh* gene derived from *Agrobacterium tumefaciens* and the *udh* gene derived from *Pseudomonas putida.*

A gene derived from a bacterium other than *Agrobacterium tumefaciens* or *Pseudomonas putida,* or from another microorganism, animal, or plant, may also be used. The gene may be, for example, a gene encoding a protein having GalADH activity, which gene is isolated from the chromosome of a microorganism, animal, plant, or the like based on homology to the *udh* gene or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GalADH activity, a plurality of types of genes derived from a different microorganism(s), animal(s), and/or plant(s) may be used.

In the present invention, the "UDP-galactose 6-dehydrogenase (hereinafter also referred to as UDP-GalDH) activity" means the activity that catalyzes a reaction to oxidize UDP-galactose to produce UDP-galacturonic acid. The "enhanced UDP-GalDH activity" means that the UDP-GalDH activity is increased compared to non-modified strains. The UDP-GalDH activity is preferably increased not less than 1.5-fold, more preferably increased not less than 3-fold, per unit microbial weight compared to non-modified strains. The fact that the UDP-GalDH activity is enhanced can be confirmed by measuring the UDP-GalDH activity by a known method such as the method by Yurist-Doutsch et al. (Mol. Microbiol., 2010, Vol. 75(4), p 1047-58).

The strain modified such that the UDP-GalDH activity is enhanced can be prepared by the same method as the above-described method for enhancing the UDP-GLE activity.

The gene to be used for the enhancement of the UDP-GalDH activity is not limited as long as it encodes a protein having UDP-GalDH activity, and examples of the gene include the *aglM* gene derived from *Haloferax volcanii.*

A gene derived from a bacterium other than *Haloferax volcanii,* or from another microorganism, animal, or plant, may also be used. The gene may be, for example, a gene encoding a protein having UDP-GalDH activity, which gene is isolated from the chromosome of a microorganism, animal, plant, or the like based on homology to the *aglM* gene or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the UDP-GalDH activity, a plurality of types of genes derived from a different microorganism(s), animal(s), and/or plant(s) may be used.

The productivity of mucic acid can be effectively increased by construction of a pathway for biosynthesis of mucic acid from UDP-glucuronic acid and, in addition, enhancement of an enzyme activity that catalyzes a rate-limiting reaction in a metabolic pathway for biosynthesis of mucic acid from glucose via UDP-glucuronic acid.

Therefore, for the microorganism of the present invention, in cases where modification is carried out such that the UDP-glucuronate 4-epimerase activity, UDP-galacturonate pyrophosphorylase activity, and galacturonate dehydrogenase activity are enhanced as compared to non-modified strains, it is preferred to further carry out modification such that at least one enzyme activity selected from the group consisting of glucokinase activity, phosphoglucomutase activity, glucose-1-phosphate uridylyltransferase activity, UDP-glucose 6-dehydrogenase activity, and galacturonate-1-phosphate phosphatase activity is enhanced as compared to non-modified strains. For further improvement of the productivity of mucic acid, the modification is preferably carried out for enhancing, among these enzyme activities, not less than two activities, more preferably not less than three activities, still more preferably not less than four activities, especially preferably all five activities.

The productivity of mucic acid can be effectively increased by construction of a pathway for biosynthesis of mucic acid from UDP-galactose and, in addition, enhancement of an enzyme activity that catalyzes a rate-limiting reaction in a metabolic pathway for biosynthesis of mucic acid from glucose or galactose via UDP-galactose.

Therefore, for the microorganism of the present invention, in cases where modification is carried out such that the UDP-galactose 6-dehydrogenase activity, UDP-galacturonate pyrophosphorylase activity, and galacturonate dehydrogenase activity are enhanced as compared to non-modified strains, it is preferred to further carry out modification such that at least one enzyme activity selected from the group consisting of glucokinase activity, phosphoglucomutase activity, glucose-1-phosphate uridylyltransferase activity, hexose-1-phosphate uridylyltransferase activity, UDP-glucose 4-epimerase activity, galactokinase activity, UDP-galactose pyrophosphorylase activity, and galacturonate-1-phosphate phosphatase activity is enhanced as compared to non-modified strains. For further improvement of the productivity of mucic acid, the modification is preferably carried out for enhancing, among these enzyme activities, not less than two or three activities, more preferably not less than four or five activities, still more preferably not less than six or seven activities, especially preferably all eight activities.

In the present invention, the "glucokinase (hereinafter also referred to as GLK) activity" means the activity that catalyzes a reaction to phosphorylate glucose to produce glucose-6-phosphate (EC:2.7.1.1, 2.7.1.2). The "enhanced GLK activity" means that the GLK activity is increased compared to non-modified strains. The GLK activity is preferably increased not less than 1.5-fold, more preferably increased not less than 3-fold, per unit microbial weight compared to non-modified strains. The fact that the GLK activity is enhanced can be confirmed by measuring the GLK activity by a known method such as the method by Wu et al. (PLoS. ONE, 2011, Vol.6(8), p e23172), the method by Fernandez et al. (J. Gen. Microbiol., 1985, Vol. 131, p 2705-2709), or the method by Meyer et al. (J. Bacteriol. Vol. 179(4), p 1298-306).

The strain modified such that the GLK activity is enhanced can be prepared by the same method as the above-described method for enhancing the UDP-GLE activity.

The *glk* gene to be used for the enhancement of the GLK activity is not limited as long as it encodes a protein having GLK activity, and examples of the gene include the *glk* gene derived from *Escherichia coli.*

A *glk* gene derived from a bacterium other than *Escherichia coli,* or from another microorganism, animal, or plant, may also be used. The *glk* gene derived from a microorganism, animal, or plant may be, for example, a gene whose base sequence has already been determined, or a gene encoding a protein having GLK activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GLK activity, a plurality of types of *glk* genes derived from a different microorganism(s), animal(s), and/or plant(s) may be used.

In the present invention, the "phosphoglucomutase (hereinafter also referred to as PGM) activity" means the activity that catalyzes a reaction to produce glucose-1-phosphate by intramolecular rearrangement of glucose-6-phosphate (EC:5.4.2.2). The "enhanced PGM activity" means that the PGM activity is increased compared to non-modified strains. The PGM activity is preferably increased not less than 1.5-fold, more preferably increased not less than 3-fold, per unit microbial weight compared to non-modified strains. The fact that the PGM activity is enhanced can be confirmed by measuring the PGM activity by a known method such as the method by Lowry et al. (J. BIOL. CHEM., 1969, Vol. 244(4), p 910-6) or the method by Walther et al. (FEBS Letters, 2012, Vol. 586(23), 4114-8).

The strain modified such that the PGM activity is enhanced can be prepared by the same method as the above-described method for enhancing the UDP-GLE activity.

The *pgm* gene to be used for the enhancement of the PGM activity is not limited as long as it encodes a protein having PGM activity, and examples of the gene include the *pgm* gene derived from *Escherichia coli.*

A *pgm* gene derived from a bacterium other than *Escherichia coli,* or from another microorganism, animal, or plant, may also be used. The *pgm* gene derived from a microorganism, animal, or plant may be, for example, a gene whose base sequence has already been determined, or a gene encoding a protein having PGM activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the PGM activity, a plurality of types of *pgm* genes derived from a different microorganism(s), animal(s), and/or plant(s) may be used.

In the present invention, the "glucose-1-phosphate uridylyltransferase (hereinafter also referred to as GalUF) activity" means the activity that catalyzes a reaction to produce UDP-glucose from UTP and glucose-1-phosphate (EC:2.7.7.9). The "enhanced GalUF activity" means that the GalUF activity is increased compared to non-modified strains. The GalUF activity is preferably increased not less than 1.5-fold, more preferably increased not less than 3-fold, per unit microbial weight compared to non-modified strains. The fact that the GalUF activity is enhanced can be confirmed by measuring the GalUF activity by a known method such as the method by Yu et al. (Biochem. J., 2012, Vol. 442(2), p 283-91) or the method by Weissobrn et al. (J. Bacteriol, Vol. 176(9), p 2611-8).

The strain modified such that the GalUF activity is enhanced can be prepared by the same method as the above-described method for enhancing the UDP-GLE activity.

The *galU* gene and the *galF* gene to be used for the enhancement of the GalUF activity are not limited as long as they encode a protein having GalUF activity, and examples of the genes include the *galU* gene and the *galF* gene derived from *Escherichia coli.*

A *galU* gene and a *galF* gene derived from a bacterium other than *Escherichia coli,* or from another microorganism, animal, or plant, may also be used. The *galU* gene and the *galF* gene derived from a microorganism, animal, or plant may be, for example, genes whose base sequences have already been determined, or genes encoding a protein having GalUF activity, which genes are isolated from the chromosome of the microorganism, animal, plant, or the like based on homology or the like followed by determination of the base sequences thereof. After the determination of the base sequences, genes synthesized according to the sequences may be used. These can be obtained by amplifying the region containing the promoter and the ORF region by the hybridization method, PCR method, or the like. For the enhancement of the GalUF activity, a plurality of types of each of the *galU* gene and the *galF* gene derived from a different microorganism(s), animal(s), and/or plant(s) may be used.

In the present invention, the "hexose-1-phosphate uridylyltransferase (hereinafter also referred to as GalT) activity" means the activity that catalyzes a reaction to produce UDP-galactose and glucose-1-phosphate from galactose-1-phosphate and UDP-glucose, or the activity that catalyzes the reverse reaction thereof (EC:2.7.7.12). The "enhanced GalT activity" means that the GalT activity is increased compared to non-modified strains. The GalT activity is preferably increased not less than 1.5-fold, more preferably increased not less than 3-fold, per unit microbial weight compared to non-modified strains. The fact that the GalT activity is enhanced can be confirmed by measuring the GalT activity by a known method such as the method by Li et al. (Clin. Chem. 2010, Vol. 56(5), p 772-80) or the method by Geeganage et al. (Biochem., 1999, Vol. 38(40), p 13398-406 or Biochem., 2000, 39(18), p 5397-404).

The strain modified such that the GalT activity is enhanced can be prepared by the same method as the above-described method for enhancing the UDP-GLE activity.

The *galT* gene to be used for the enhancement of the GalT activity is not limited as long as it encodes a protein having GalT activity, and examples of the gene include the *galT* gene derived from *Escherichia coli.*

A *galT* gene derived from a bacterium other than *Escherichia coli,* or from another microorganism, animal, or plant, may also be used. The *galT* gene derived from a microorganism, animal, or plant may be, for example, a gene whose base sequence has already been determined, or a gene encoding a protein having GalT activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GalT activity, a plurality of types of *galT* genes derived from a different microorganism(s), animal(s), and/or plant(s) may be used.

In the present invention, the "UDP-glucose 6-dehydrogenase (hereinafter also referred to as UGD) activity" means the activity that catalyzes a reaction to oxidize UDP-glucose to produce UDP-glucuronic acid (EC:1.1.1.22). The "enhanced UGD activity" means that the UGD activity is increased compared to non-modified strains. The UGD activity is preferably increased not less than 1.5-fold, more preferably increased not less than 3-fold, per unit microbial weight compared to non-modified strains. The fact that the UGD activity is enhanced can be confirmed by measuring the UGD activity by a known method such as the method by Pagni et al. (Microbiol., 1999, Vol. 145, p 1049-53), the method by Sennet et al. (Biochem., 2012, Vol. 51(46), p 9364-74), or Kadirvelraj (Biochem., 2013, Vol. 52(8), p 1456-65).

The strain modified such that the UGD activity is enhanced can be prepared by the same method as the above-described method for enhancing the UDP-GLE activity.

The *ugd* gene to be used for the enhancement of the UGD activity is not limited as long as it encodes a protein having UGD activity, and examples of the gene include the *ugd* gene derived from *Escherichia coli.*

A *ugd* gene derived from a bacterium other than *Escherichia coli,* or from another microorganism, animal, or plant, may also be used. The *ugd* gene derived from a microorganism, animal, or plant may be, for example, a gene whose base sequence has already been determined, or a gene encoding a protein having UGD activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the UGD activity, a plurality of types of *ugd* genes derived from a different microorganism(s), animal(s), and/or plant(s) may be used.

In the present invention, the "UDP-glucose 4-epimerase (hereinafter also referred to as GalE) activity" means the activity that catalyzes a reaction to isomerize UDP-glucose to produce UDP-galactose (EC:5.1.3.2). The "enhanced GalE activity" means that the GalE activity is increased compared to non-modified strains. The GalE activity is preferably increased not less than 1.5-fold, more preferably increased not less than 3-fold, per unit microbial weight compared to non-modified strains. The fact that the GalE activity is enhanced can be confirmed by measuring the GalE activity by a known method such as the method by Daenzer et al. (PLoS. Genet., 2012, Vol. 8(5), p e1002721) or the method by Kim et al. (Appl. Biochem. Biotechnol., 2011, Vol. 163(3), p 444-51).

The strain modified such that the GalE activity is enhanced can be prepared by the same method as the above-described method for enhancing the UDP-GLE activity.

The *galE* gene to be used for the enhancement of the GalE activity is not limited as long as it encodes a protein having GalE activity, and examples of the gene include the *galE* gene derived from *Escherichia coli.*

A *galE* gene derived from a bacterium other than *Escherichia coli,* or from another microorganism, animal, or plant, may also be used. The *galE* gene derived from a microorganism, animal, or plant may be, for example, a gene whose base sequence has already been determined, or a gene encoding a protein having GalE activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GalE activity, a plurality of types of *galE* genes derived from a different microorganism(s), animal(s), and/or plant(s) may be used.

In the present invention, the "galactokinase (hereinafter also referred to as GalK) activity" means the activity that catalyzes a reaction to phosphorylate galactose to produce galactose-1-phosphate (EC:2.7.1.6). The "enhanced GalK activity" means that the GalK activity is increased compared to non-modified strains. The GalK activity is preferably increased not less than 1.5-fold, more preferably increased not less than 3-fold, per unit microbial weight compared to non-modified strains. The fact that the GalK activity is enhanced can be confirmed by measuring the GalK activity by a known method such as the method by Tedesco et al. (J. Clin. Invest., 1969, Vol. 48(12), p 2390-7) or the method by Hoffmeister et al. (ChemBioChem, 2004 Vol. 5(7), p 989-92).

The strain modified such that the GalK activity is enhanced can be prepared by the same method as the above-described method for enhancing the UDP-GLE activity.

The *galK* gene to be used for the enhancement of the GalK activity is not limited as long as it encodes a protein having GalK activity, and examples of the gene include the *galK* gene derived from *Escherichia coli.*

A *galK* gene derived from a bacterium other than *Escherichia coli,* or from another microorganism, animal, or plant, may also be used. The *galK* gene derived from a microorganism, animal, or plant may be, for example, a gene whose base sequence has already been determined, or a gene encoding a protein having GalK activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GalK activity, a plurality of types of *gal*K genes derived from a different microorganism(s), animal(s), and/or plant(s) may be used.

In the present invention, the "UDP-galactose pyrophosphorylase (hereinafter also referred to as UDP-GalPPase) activity" means the activity that catalyzes a reaction to produce UDP-galactose from UTP or TTP and galactose-1-phosphate (EC:2.7.7.10, 2.7.7.64). The "enhanced UDP-GalPPase activity" means that the UDP-GalPPase activity is increased compared to non-modified strains. The UDP-GalPPase activity is preferably increased not less than 1.5-fold, more preferably increased not less than 3-fold, per unit microbial weight compared to non-modified strains. The fact that the UDP-GalPPase activity is enhanced can be confirmed by measuring the UDP-GalPPase activity by a known method such as the method by Ebrecht et al. (Biochimica et Biophysica Acta., 2015, Vol. 1850(1), p 88-96) or the method by Damerow et al. (J. Biol. Chem., 2010, Vol. 285(2), p 878-87).

The strain modified such that the UDP-GalPPase activity is enhanced can be prepared by the same method as the above-described method for enhancing the UDP-GLE activity.

The *ugp* gene to be used for the enhancement of the UDP-GalPPase activity is not limited as long as it encodes a protein having UDP-GalPPase activity, and examples of the gene include the *ugp* gene derived from *Giardia lamblia.*

A *ugp* gene derived from a protozoan other than *Giardia lamblia,* or from another microorganism, animal, or plant, may also be used. The *ugp* gene derived from a microorganism, animal, or plant may be, for example, a gene whose base sequence has already been determined, or a gene encoding a protein having UDP-GalPPase activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the UDP-GalPPase activity, a plurality of types of *ugp* genes derived from a different microorganism(s), animal(s), and/or plant(s) may be used.

In the present invention, the "galacturonate-1-phosphate phosphatase (hereinafter also referred to as GalA1P) activity" means the activity that catalyzes a reaction to dephosphorylate galacturonate-1-phosphate to produce galacturonic acid. The "enhanced GalAlP activity" means that the GalAlP activity is increased compared to non-modified strains. The GalAlP activity is preferably increased not less than 1.5-fold, more preferably increased not less than 3-fold, per unit microbial weight compared to non-modified strains. The fact that the GalAlP activity is enhanced can be confirmed by measuring the GalAlP activity by a known method such as the method by Passariello et al. (Biochimica et Biophysica Acta, 2006, Vol. 1764, p 13-9) or the method by Dassa et al. (J. BIOL. CHEM., 1982, Vol. 257(12), p 6669-76).

The strain modified such that the GalAlP activity is enhanced can be prepared by the same method as the above-described method for enhancing the UDP-GLE activity.

The phosphatase gene to be used for the enhancement of the GalAlP activity is not limited as long as it encodes a protein having GalAlP activity, and examples of the gene include the *agp* gene and the aphA gene derived from *Escherichia coli.*

A phosphatase gene derived from a bacterium other than *Escherichia coli,* or from another microorganism, animal, or plant, may also be used. The phosphatase gene derived from a microorganism, animal, or plant may be, for example, a gene whose base sequence has already been determined, or a gene encoding a protein having GalAlP activity, which gene is isolated from the chromosome of the microorganism, animal, plant, or the like based on homology or the like followed by determination of the base sequence thereof. After the determination of the base sequence, a gene synthesized according to the sequence may be used. It can be obtained by amplifying the region containing the promoter and the ORF region by the hybridization method, PCR method, or the like. For the enhancement of the GalAlP activity, a plurality of types of phosphatase genes derived from a different microorganism(s), animal(s), and/or plant(s) may be used.

The phosphatase gene used preferably has a mutation(s) that was/were introduced for improvement of the substrate specificity. In cases of an *agp* gene derived from *Escherichia coli,* the substrate specificity can be improved by introducing mutations for changing amino acid residues such that the arginine at position 94 is changed to histidine; the methionine at position 123 is changed to valine or alanine; and the glutamic acid at position 196 is changed to aspartic acid. These mutations may be introduced in combination.

The productivity of mucic acid can be effectively increased by construction of a pathway for biosynthesis of mucic acid from UDP-glucuronic acid and, in addition, reduction of an enzyme activity that catalyzes a reaction which competes with the metabolic pathway for biosynthesis of mucic acid from glucose via UDP-glucuronic acid, and which produces a by-product.

Thus, the microorganism of the present invention is preferably a microorganism which is modified to have enhanced UDP-glucuronate 4-epimerase activity, UDP-galacturonate pyrophosphorylase activity, and galacturonate dehydrogenase activity, as compared to non-modified strains, and to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, hexose-1-phosphate uridylyltransferase activity, UDP-glucose 4-epimerase activity, UDP-glucuronic acid dehydrogenase activity, galacturonate isomerase activity, galacturonate reductase activity, and galactarate dehydratase activity, as compared to non-modified strains. For further improvement of the productivity of mucic acid, the modification may be carried out for decreasing, among these enzyme activities, preferably not less than two or not less than three activities, more preferably not less than four or not less than five activities, still more preferably not less than six or not less than seven activities, especially preferably not less than eight or not less than nine activities, most preferably all ten activities.

For further improvement of the productivity of mucic acid, it is preferred to combine these modifications with a modification that enhances an enzyme activity that catalyzes a rate-limiting reaction in the metabolic pathway for biosynthesis of mucic acid from glucose via UDP-glucuronic acid. In cases where such a plurality of modifications are carried out, they may be carried out in an arbitrary order.

The productivity of mucic acid can be effectively increased by construction of a pathway for biosynthesis of mucic acid from UDP-galactose and, in addition, reduction of an enzyme activity that catalyzes a reaction which competes with the metabolic pathway for biosynthesis of mucic acid from glucose or galactose via UDP-galactose, and which produces a by-product.

Thus, the microorganism of the present invention is preferably a microorganism which is modified to have enhanced UDP-galactose 6-dehydrogenase activity, UDP-galacturonate pyrophosphorylase activity, and galacturonate dehydrogenase activity, as compared to non-modified strains, and to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, UDP-glucose 6-dehydrogenase activity, UDP-glucuronic acid dehydrogenase activity, galacturonate isomerase activity, galacturonate reductase activity, and galactarate dehydratase activity, as compared to non-modified strains. For further improvement of the productivity of mucic acid, the modification may be carried out for enhancing, among these enzyme activities, preferably not less than two activities, more preferably not less than three or not less than four activities, still more preferably not less than five or not less than six activities, especially preferably not less than seven or not less than eight activities, most preferably all nine activities.

For further improve ement of the productivity of mucic acid, it is preferred to combine these modifications with a modification that enhances an enzyme activity that catalyzes a rate-limiting reaction in the metabolic pathway for biosynthesis of mucic acid from glucose or galactose via UDP-galactose. In cases where such a plurality of modifications are carried out, they may be carried out in an arbitrary order.

In the present invention, the "glucose-6-phosphate 1-dehydrogenase (hereinafter also referred to as ZWF) activity" means the activity that catalyzes a reaction to dehydrogenate glucose-6-phosphate to produce 6-phosphoglucono-1,5-lactone (EC:1.1.1.49, 1.1.1.363, 1.1.1.388). The "decreased ZWF activity" means that the ZWF activity is decreased compared to non-modified strains. The ZWF activity is preferably decreased to not more than 30%, more preferably decreased to not more than 10%, per unit microbial weight compared to non-modified strains. The ZWF activity may be completely eliminated. The fact that the ZWF activity is decreased can be confirmed by measuring the ZWF activity by a known method such as the method by Ma et al. (J. Bacteriol., 1998, Vol. 180(7), p 1741-9) or the method by Banerjee et al. (J. Bacteriol., 1972, Vol. 110(1), p 155-60).

The strain having decreased ZWF activity can be obtained by using the above-described microorganism as a parent strain, and treating the parent strain with a mutagen ordinarily used for mutation treatment, such as N-methyl-N'-nitro-N-nitrosoguanidine (NTG) or ethyl methanesulfonate (EMS), followed by selecting a strain having decreased ZWF activity. This can be also be achieved by, for example, destroying the *zwf* gene on the chromosome or modifying an expression regulatory sequence such as the promoter or the Shine-Dalgarno (SD) sequence. More specifically, for example, a region of the zwf gene on the chromosome including the promoter sequence may be partially or entirely deleted; a base sequence may be inserted into the region to disrupt the region; a mutation(s) may be introduced to the region; or the promoter may be replaced with a weaker promoter. A plurality of these methods for decreasing the ZWF activity may be used in combination.

Examples of the method for preparing the strain modified to have decreased ZWF activity include a method in which homologous recombination of the chromosome is carried out (see JP 11-206385 A or the like) and a method in which the *sacB* gene is used (Gene, 1994, Vol. 145(1), p 69-73).

In the present invention, the "glucose-6-phosphate isomerase (hereinafter also referred to as PGI) activity" means the activity that catalyzes a reaction to isomerize glucose-6-phosphate to produce fructose-6-phosphate (EC:5.3.1.9). The "decreased PGI activity" means that the PGI activity is decreased compared to non-modified strains. The PGI activity is preferably decreased to not more than 30%, more preferably decreased to not more than 10%, per unit microbial weight compared to non-modified strains. The PGI activity may be completely eliminated. The fact that the PGI activity is decreased can be confirmed by measuring the PGI activity by a known method such as the method by Horrocks et al. (J. Clin. Pathol., 1963, Vol. 16, p 248-51) or the method by Milewski et al. (Arch. Biochem. Biophys., 2006, Vol. 450(1), p 39-49).

The strain modified such that the PGI activity is decreased can be prepared by the same method as the above-described method for decreasing the ZWF activity.

In the present invention, the "glucose-1-phosphate phosphatase (hereinafter also referred to as AGP) activity" means the activity that catalyzes a reaction to dephosphorylate glucose-1-phosphate to produce glucose (EC:3.1.3.10). The "decreased AGP activity" means that the AGP activity is decreased compared to non-modified strains. The AGP activity is preferably decreased to not more than 30%, more preferably decreased to not more than 10%, per unit microbial weight compared to non-modified strains. The AGP activity may be completely eliminated. The fact that the AGP activity is decreased can be confirmed by measuring the AGP activity by a known method such as the method by Pradel et al. (J. Bacteriol., 1988, Vol. 170(10), p 4916-23).

The strain modified such that the AGP activity is decreased can be prepared by the same method as the above-described method for decreasing the ZWF activity.

In the present invention, the "ADP-glucose pyrophosphorylase (hereinafter also referred to as AGPase) activity" means the activity that catalyzes a reaction to produce ADP-glucose from glucose-1-phosphate and ATP (EC:2.7.7.27). The "decreased AGPase activity" means that the AGPase activity is decreased compared to non-modified strains. The AGPase activity is preferably decreased to not more than 30%, more preferably decreased to not more than 10%, per unit microbial weight compared to non-modified strains. The AGPase activity may be completely eliminated. The fact that the AGPase activity is decreased can be confirmed by measuring the AGPase activity by a known method such as the method by Seibold et al. (Microbiology, 2007, Vol. 153(Pt4), p 1275-85).

The strain modified such that the AGPase activity is decreased can be prepared by the same method as the above-described method for decreasing the ZWF activity.

In the present invention, the "hexose-1-phosphate uridylyltransferase (hereinafter also referred to as GalT) activity" means the activity that catalyzes a reaction to produce UDP-galactose and glucose-1-phosphate from galactose-1-phosphate and UDP-glucose, or the activity that catalyzes the reverse reaction thereof (EC:2.7.7.12). The "decreased GalT activity" means that the GalT activity is decreased compared to non-modified strains. The PGI activity is preferably decreased to not more than 30%, more preferably decreased to not more than 10%, per unit microbial weight compared to non-modified strains. The GalT activity may be completely eliminated. The fact that the GalT activity is decreased can be confirmed by measuring the GalT activity by a known method such as the method by Saito et al. (J. Biol. Chem., 1967, Vol. 242(10), p 2362-8) or the method by Davit-Spraul (FEBS Lett., 1994, Vol. 354, p 232-6).

The strain modified such that the GalT activity is decreased can be prepared by the same method as the above-described method for decreasing the ZWF activity.

In the present invention, the "UDP-glucose 4-epimerase (hereinafter also referred to as GalE) activity" means the activity that catalyzes a reaction to isomerize UDP-glucose to produce UDP-galactose (EC:5.1.3.2). The "decreased GalE activity" means that the GalE activity is decreased compared to non-modified strains. The GalE activity is preferably decreased to not more than 30%, more preferably decreased to not more than 10%, per unit microbial weight compared to non-modified strains. The GalE activity may be completely eliminated. The fact that the GalE activity is decreased can be confirmed by measuring the GalE activity by a known method such as the method by Kotake et al. (Biochem. J., 2009, Vol. 424(2) p 169-77) or the method by Schulz et al. (J Biol Chem., 2005, Vol. 280(14), p 13493-502).

The strain modified such that the GalE activity is decreased can be prepared by the same method as the above-described method for decreasing the ZWF activity.

In the present invention, the "UDP-glucose 6-dehydrogenase (hereinafter also referred to as UGD) activity" means the activity that catalyzes a reaction to oxidize UDP-glucose to produce UDP-glucuronic acid (EC:1.1.1.22). The "decreased UGD activity" means that the UGD activity is decreased compared to non-modified strains. The UGD activity is preferably decreased to not more than 30%, more preferably decreased to not more than 10%, per unit microbial weight compared to non-modified strains. The UGD activity may be completely eliminated. The fact that the UGD activity is decreased can be confirmed by measuring the UGD activity by a known method such as the method by Easley et al. (Biochem., 2007, Vol. 46(2), p 369-378), the method by Sennet et al. (Biochem., 2012, Vol. 51(46), p 9364-74), or Kadirvelraj (Biochem., 2013, Vol. 52(8), p 1456-65).

The strain modified such that the UGD activity is decreased can be prepared by the same method as the above-described method for decreasing the ZWF activity.

In the present invention, the "UDP-glucuronic acid dehydrogenase (hereinafter also referred to as ArnA) activity" means the activity that catalyzes a reaction to oxidatively decarboxylate UDP-glucuronic acid to produce UDP-4-keto-arabinose (EC:1.1.1.305, 2.1.2.13). The "decreased ArnA activity" means that the ArnA activity is decreased compared to non-modified strains. The ArnA activity is preferably decreased to not more than 30%, more preferably decreased to not more than 10%, per unit microbial weight compared to non-modified strains. The ArnA activity may be completely eliminated. The fact that the ArnA activity is decreased can be confirmed by measuring the ArnA activity by a known method such as the method by Gatzeva-Topalova et al. (Biochemistry, 2004, Vol. 43(42), p 13370-9).

The strain modified such that the ArnA activity is decreased can be prepared by the same method as the above-described method for decreasing the ZWF activity.

In the present invention, the "galacturonate isomerase (hereinafter also referred to as UxaC) activity" means the activity that catalyzes a reaction to isomerize galacturonic acid to produce tagaturonic acid (EC:5.3.1.12). The "decreased UxaC activity" means that the UxaC activity is decreased compared to non-modified strains. The UxaC activity is preferably decreased to not more than 30%, more preferably decreased to not more than 10%, per unit microbial weight compared to non-modified strains. The UxaC activity may be completely eliminated. The fact that the UxaC activity is decreased can be confirmed by measuring the UxaC activity by a known method such as the method by Portalier et al. (Mol. Gen. Genet., 1974, Vol. 128(4), p 301-19).

The strain modified such that the UxaC activity is decreased can be prepared by the same method as the above-described method for decreasing the ZWF activity.

In the present invention, the "galacturonate reductase (hereinafter also referred to as GalUR) activity" means the activity that catalyzes a reaction to reduce galacturonic acid to produce galactonic acid (EC:1.1.1.365). The "decreased GalUR activity" means that the GalUR activity is decreased compared to non-modified strains. The GalUR activity is preferably decreased to not more than 30%, more preferably decreased to not more than 10%, per unit microbial weight compared to non-modified strains. The GalUR activity may be completely eliminated. The fact that the GalUR activity is decreased can be confirmed by measuring the GalUR activity by a known method such as the method by Martens-Uzunova et al. (Fungal Genet. Biol., 2008, Vol. 45(11), p 1449-57) or Zhang (Fungal Genet. Biol., 2011, Vol. 48(10), p 990-7).

The strain modified such that the GalUR activity is decreased can be prepared by the same method as the above-described method for decreasing the ZWF activity.

In the present invention, the "galactarate dehydratase (hereinafter also referred to as GarD) activity" means the activity that catalyzes a reaction to dehydrate mucic acid to produce (2R,3S)-2,3-dihydroxy-5-oxohexanedioic acid (EC:4.2.1.42). The "decreased GarD activity" means that the GarD activity is decreased compared to non-modified strains. The GarD activity is preferably decreased to not more than 30%, more preferably decreased to not more than 10%, per unit microbial weight compared to non-modified strains. The GarD activity may be completely eliminated. The fact that the GarD activity is decreased can be confirmed by measuring the GarD activity by a known method such as the method by Hubbard et al. (Biochemistry, 1998, Vol. 37(41), p 14369-75) or the method by Wen et al. (Biochem., 2007, 46(33), p 9564-77).

The strain modified such that the GarD activity is decreased can be prepared by the same method as the above-described method for decreasing the ZWF activity.

### <Second Method for Producing Mucic Acid>

The second method for producing mucic acid of the present invention comprises a step of allowing the microorganism of the present invention or a treated product thereof to act on an organic raw material in an aqueous medium to produce mucic acid (hereinafter referred to as "fermentation step"). The second method for producing mucic acid of the present invention is a mode preferred for carrying out the first method for producing mucic acid of the present invention.

In the second method for producing mucic acid of the present invention, a treated product of microbial cells of the microorganism of the present invention may also be used. Examples of the treated product of the microorganism include fixed microbial cells prepared by fixation of microbial cells of the microorganism with acrylamide, carrageenan, or the like; a homogenate prepared by homogenization of microbial cells; a supernatant obtained by centrifugation of the homogenate; and a fraction obtained by partial purification of the supernatant by ammonium sulfate treatment or the like. These treated products normally contain various enzymes involved in biosynthesis of mucic acid, retained by the microorganism of the present invention.

The second method for producing mucic acid of the present invention preferably comprises a step of recovering the produced mucic acid after the fermentation step (hereinafter referred to as "recovery step").

When the microorganism of the present invention is used in the second method for producing mucic acid of the present invention, cultured cells obtained by slant culture using a solid medium such as agar medium may be directly used. Alternatively, prior to the fermentation step, the microorganism may be preliminarily cultured in a liquid medium, if necessary. That is, the fermentation step may be carried out after growing the microorganism of the present invention by performing the later-described seed culture and/or main culture.

The later-described seed culture and main culture may also be carried out at the same time as, rather than separately from, the later-described fermentation step. The production of mucic acid may also be carried out by allowing the microorganism grown by the seed culture or the main culture to react with an organic raw material while allowing the microorganism to grow in the reaction liquid.

### (Seed Culture)

The seed culture is carried out for preparation of the microbial cells of the microorganism of the present invention to be subjected to the main culture. The medium to be used for the seed culture may be an ordinary medium that is used for culture of microorganisms. The medium is preferably a medium containing a nitrogen source, inorganic salt, and/or the like. The nitrogen source is not limited as long as it is a nitrogen source that can be assimilated by the microorganism of the present invention to allow the growth of the microorganism. Specific examples of the nitrogen source include various organic and inorganic nitrogen compounds such as ammonium salts, nitric acid salts, urea, soybean hydrolysates, casein digests, peptone, yeast extracts, meat extracts, and corn steep liquor. Examples of the inorganic salt include phosphoric acid salts; sulfuric acid salts; and salts of metals such as magnesium, potassium, manganese, iron, and zinc. If necessary, a factor that promotes the growth is added. Examples of such a factor include vitamins such as biotin, thiamine, pantothenic acid, inositol, and nicotinic acid; nucleotides; and amino acids.

In the seed culture, if necessary, a carbon source may be added to the medium. The carbon source to be used for the seed culture is not limited as long as it can be assimilated by the microorganism to allow the growth of the microorganism. Examples of the carbon sources that are usually used include carbohydrates such as glucose, fructose, galactose, lactose, xylose, arabinose, sucrose, starch, and cellulose; and fermentable carbohydrates such as polyalcohols including glycerol, mannitol, inositol, and ribitol. Among these, glucose, fructose, galactose, lactose, sucrose, xylose, and arabinose are preferred. Glucose, galactose, sucrose, and lactose are especially preferred. These carbon sources may be added individually, or may be added in combination.

The seed culture is preferably carried out at a common optimum growth temperature. The common optimum growth temperature means the temperature at which the growth rate is highest under the conditions used for the production of mucic acid. More specifically, the culture temperature is usually 25°C to 40°C, preferably 30°C to 37°C. In cases of coliform bacteria, the culture temperature is usually 25°C to 40°C, more preferably 30°C to 38°C, especially preferably about 37°C. In cases of coryneform bacteria, the culture temperature is usually 25°C to 35°C, more preferably 28°C to 33°C, especially preferably about 30°C. In cases of yeasts, the culture temperature is usually 25°C to 40°C, more preferably 28°C to 35°C, especially preferably about 30°C.

The seed culture is preferably carried out at a common optimum growth pH. The common optimum growth pH means the pH at which the growth rate is highest under the conditions used for the production of mucic acid. More specifically, the culture pH is usually pH 3 to 10, preferably pH 5 to 8. In cases of coliform bacteria, the culture pH is usually pH 5 to 8.5, preferably pH 6 to 8. In cases of coryneform bacteria, the culture pH is usually pH 6 to 9, preferably pH 6.5 to 8.5. In cases of yeasts, the culture pH is usually pH 3 to 8, preferably pH 4.5 to 7.5.

The culture period of the seed culture is not limited as long as it is a period in which a certain amount of microbial cells are obtained. The culture period is usually 6 hours to 96 hours. In the seed culture, it is preferred to supply oxygen by aeration, stirring, and/or the like.

The microbial cells after the seed culture can be used for the later-described main culture. The seed culture may be omitted, and cultured cells obtained by slant culture using a solid medium such as agar medium may be directly used for the main culture. If necessary, the seed culture may be carried out repeatedly several times.

### (Main Culture)

The main culture is carried out for preparation of the microbial cells of the microorganism of the present invention to be subjected to the later-described mucic acid-producing reaction, and its main purpose is to increase the amount of the microbial cells. When the seed culture described above is carried out, the microbial cells obtained by the seed culture are used to perform the main culture.

The medium to be used for the main culture may be an ordinary medium that is used for culture of microorganisms. The medium is preferably a medium containing a nitrogen source, inorganic salt, and/or the like. The nitrogen source herein is not limited as long as it is a nitrogen source that can be assimilated by the microorganism to allow the growth of the microorganism. Specific examples of the nitrogen source include various organic and inorganic nitrogen compounds such as ammonium salts, nitric acid salts, urea, soybean hydrolysates, casein digests, peptone, yeast extracts, meat extracts, and corn steep liquor. Examples of the inorganic salt include phosphoric acid salts; sulfuric acid salts; and salts of metals such as magnesium, potassium, manganese, iron, and zinc. If necessary, a factor that promotes the growth is added. Examples of such a factor include vitamins such as biotin, thiamine, pantothenic acid, inositol, and nicotinic acid; nucleotides; and amino acids. For suppressing foaming during the culture, the medium is preferably supplemented with an appropriate amount of a commercially available antifoaming agent.

In the main culture, a carbon source is preferably added to the medium. The carbon source to be used for the main culture is not limited as long as it can be assimilated by the microorganism to allow the growth of the microorganism. Examples of the carbon sources that are usually used include carbohydrates such as glucose, fructose, galactose, lactose, xylose, arabinose, sucrose, starch, and cellulose; and fermentable carbohydrates such as polyalcohols including glycerol, mannitol, inositol, and ribitol. Among these, glucose, fructose, galactose, lactose, sucrose, xylose, and arabinose are preferred. Glucose, galactose, sucrose, and lactose are especially preferred. These carbon sources may be added individually, or may be added in combination.

A starch saccharified liquid, molasses, or the like containing the fermentable carbohydrate may also be used. In this case, the fermentable carbohydrate is preferably a sugar liquid obtained by squeezing a plant such as sugar cane, sugar beet, or sugar maple.

These carbon sources may be added individually, or may be added in combination.

The concentration at which the carbon source is used is not limited. It is advantageous to add the carbon source in an amount which does not inhibit the growth of the microorganism. The carbon source may be used usually within the range of 0.1 to 10% (w/v), preferably 0.5 to 5% (w/v). The carbon source may be further added as the carbon source decreases due to the growth.

The main culture is preferably carried out at a common optimum growth temperature. More specifically, the culture temperature is usually 25°C to 40°C, preferably 30°C to 37°C. In cases of coliform bacteria, the culture temperature is usually 25°C to 40°C, more preferably 30°C to 38°C, especially preferably about 37°C. In cases of coryneform bacteria, the culture temperature is usually 25°C to 35°C, more preferably 28°C to 33°C, especially preferably about 30°C. In cases of yeasts, the culture temperature is usually 25°C to 40°C, more preferably 28°C to 35°C, especially preferably about 30°C.

The main culture is preferably carried out at a common optimum growth pH. More specifically, the culture pH is usually pH 4 to 10, preferably pH 6 to 8. In cases of coliform bacteria, the culture pH is usually pH 5 to 8.5, preferably pH 6 to 8. In cases of coryneform bacteria, the culture pH is usually pH 6 to 9, preferably pH 6.5 to 8.5. In cases of yeasts, the culture pH is usually pH 3 to 8, preferably pH 4.5 to 7.5.

The culture period of the main culture is not limited as long as it is a period in which a certain amount of microbial cells are obtained. The culture period is usually 6 hours to 96 hours. In the main culture, it is preferred to supply oxygen by aeration, stirring, and/or the like.

As a method for preparing microbial cells more suitable for the production of mucic acid, the main culture may be carried out by the method described in JP 2008-259451 A, in which depletion and satisfaction of the carbon source is alternately repeated in a short time.

The microbial cells after the main culture can be used for the later-described mucic acid-producing reaction. The culture liquid may be directly used, or microbial cells after recovery by centrifugation, membrane separation, and/or the like may be used.

### (Fermentation Step)

In the fermentation step, the microorganism having mucic acid-producing ability or a treated product thereof is allowed to act on an organic raw material in an aqueous medium to produce mucic acid. The reaction which occurs in this fermentation step is hereinafter referred to as the "mucic acid-producing reaction".

The aqueous medium herein means an aqueous solution for carrying out the mucic acid-producing reaction in the fermentation step. The aqueous medium is preferably an aqueous solution containing a nitrogen source, inorganic salt, and/or the like as described later. By reacting the microorganism of the present invention or a treated product thereof with the organic raw material in the aqueous medium, the mucic acid-producing reaction can be carried out. In the present description, the aqueous medium means the entire liquid contained in the reaction vessel.

The aqueous medium may be, for example, either a medium for culture of microorganisms, or a buffer such as phosphate buffer. The reaction liquid is preferably an aqueous solution containing a nitrogen source, inorganic salt, and/or the like. The nitrogen source herein is not limited as long as it is a nitrogen source that can be assimilated by the microorganism of the present invention to allow production of mucic acid. Specific examples of the nitrogen source include various organic and inorganic nitrogen compounds such as ammonium salts, nitric acid salts, urea, soybean hydrolysates, casein digests, peptone, yeast extracts, meat extracts, and corn steep liquor. Examples of the inorganic salt include phosphoric acid salts; sulfuric acid salts; and salts of metals such as magnesium, potassium, manganese, iron, and zinc. If necessary, a factor that promotes the growth is added. Examples of such a factor include vitamins such as biotin, thiamine, pantothenic acid, inositol, and nicotinic acid; nucleotides; and amino acids. For suppressing foaming during the reaction, the reaction liquid is preferably supplemented with an appropriate amount of a commercially available antifoaming agent.

The organic raw material to be used for the second method for producing mucic acid of the present invention is not limited as long as it can be assimilated by the microorganism of the present invention to allow production of mucic acid. The organic raw material may be the so-called carbohydrate which is commonly used.

Specific examples of the organic raw material include monosaccharides having three carbon atoms (trioses) such as glyceraldehyde; monosaccharides having four carbon atoms (tetroses) such as erythrose, threose, and erythrulose; monosaccharides having five carbon atoms (pentoses) such as ribose, lyxose, xylose, arabinose, deoxyribose, xylulose, and ribulose; monosaccharides having six carbon atoms (hexoses) such as allose, talose, gulose, glucose, altrose, mannose, galactose, idose, fucose, fuculose, rhamnose, psicose, fructose, sorbose, and tagatose; monosaccharides having seven carbon atoms (heptoses) such as sedoheptulose; disaccharides such as sucrose, lactose, maltose, trehalose, turanose, and cellobiose; trisaccharides such as raffinose, melezitose, and maltotriose; oligosaccharides such as fructo-oligosaccharides, galacto-oligosaccharides, and manna-oligosaccharides; polysaccharides such as starch, dextrin, cellulose, hemicellulose, glucan, and pentosan; and polyalcohols such as glycerol, mannitol, inositol, and ribitol.

Among the carbohydrates described above, carbohydrates containing, as constituents, monosaccharides having three to seven carbon atoms are preferred. Among these, at least one selected from the group consisting of hexoses; pentoses; and disaccharides containing these as constituents; is more preferred.

Glucose, fructose, mannose, and galactose are preferred as the hexoses. Glucose and galactose are more preferred. Xylose and arabinose are preferred as the pentoses. Xylose is more preferred. Sucrose and lactose are preferred as the disaccharides containing hexoses and pentoses as constituents. This is because glucose, galactose, xylose, sucrose, and lactose are major constituents of plants in nature, and therefore the raw material is easily available.

The organic raw material used in the second method for producing mucic acid of the present invention may contain only one kind of sugar, or may contain two or more kinds of sugars.

The organic raw material to be used for the second method for producing mucic acid of the present invention is not limited as long as it contains the above-described carbohydrate(s). Examples of the organic raw material include aqueous solutions prepared by dissolving one or more kinds of the carbohydrates described above in water; products prepared by decomposing, to a carbohydrate(s), a plant body or a part thereof containing one or more kinds of the carbohydrates described above as a constituent(s); and products prepared by extracting a carbohydrate(s) from a plant body or a part thereof containing one or more kinds of the carbohydrates described above as a constituent(s). Specific examples of the organic raw material include the later-described lignocellulose-decomposed raw materials, sucrose-containing raw materials, and starch-decomposed raw materials.

If necessary, the organic raw material to be used for the second method for producing mucic acid of the present invention may be diluted with water or the like to decrease the carbohydrate concentration, or may be concentrated to increase the carbohydrate concentration.

The concentration of the carbohydrate(s) contained in the organic raw material in the second method for producing mucic acid of the present invention is not limited since it largely varies depending on the origin of the organic raw material, the type(s) of the carbohydrate(s) contained, and the like. Taking into account the productivities in the fermentation production process and the chemical conversion process, the concentration is usually not less than 0.1% by mass, preferably not less than 2% by mass, and usually not more than 80% by mass, preferably not more than 70% by mass. In cases two or more kinds of carbohydrates are contained, the above concentration represents the total concentration.

Preferred examples of the organic raw material include lignocellulose-decomposed raw materials.

Lignocellulose is an organic matter constituted by cellulose and hemicellulose, which are structural polysaccharides, and lignin, which is an aromatic compound polymer. Lignocellulose is usually inedible, and usually discarded or incinerated in most cases. Thus, it can be stably supplied, and enables effective use of the resource, which is preferred.

Examples of lignocellulose-decomposed raw materials that can be preferably used include herbaceous biomasses such as bagasse, corn stover, wheat straw, rice straw, switchgrass, napier grass, *Erianthus,* bamboo grass (sasa), and Japanese pampas grass (susuki); and woody biomasses such as waste wood, sawdust, bark, and waste paper. In particular, bagasse, corn stover, and wheat straw are preferred.

The method for obtaining the organic raw material from the lignocellulose-decomposed raw material is not limited, and examples of the method include a method in which lignocellulose is pretreated, if necessary, and then subjected to hydrolysis using an enzyme, acid, subcritical water, supercritical water, or the like, or to thermal decomposition.

Preferred examples of the organic raw material also include sucrose-containing raw materials.

Sucrose is contained in plants capable of accumulating sucrose in the cell. Such plants are hereinafter referred to as "sucrose-containing plants". Examples of the sucrose-containing plants include those used as raw materials of sugar, such as sugar cane, sugar beet, sugar maple, palmyra palm, and sorghum. In particular, sugar cane and sugar beet are preferred.

The method for obtaining the organic raw material from the sucrose-containing plant is not limited, and examples of the method include a method in which the plant is pulverized followed by squeezing or leaching. In the second method for producing mucic acid of the present invention, the thus obtained juice (for example, cane juice in the case of sugar cane), raw sugar, waste molasses, or the like of the sucrose-containing plant may also be used as the organic raw material.

Preferred examples of the organic raw material also include starch-decomposed raw materials.

Starch is contained in plants capable of accumulating starch in the cell. Such plants are hereinafter referred to as "starch-containing plants". Examples of starch-containing plants include cassava, maize, potato, wheat, sweet potato, sago palm, rice, kudzu, dogtooth violet (katakuri), mung bean, bracken (warabi), and *Cardiocrinum cordatum* var. *glehnii* (ooubayuri). In particular, cassava, maize, potato, and wheat are preferred.

The method for obtaining the organic raw material from the starch-containing plant is not limited, and examples of the method include a method in which starch extracted from the plant is hydrolyzed.

The concentration of the organic raw material used is not limited. From the viewpoint of advantage in the productivity, the concentration is preferably as high as possible as long as the production of mucic acid is not inhibited. The concentration of the organic raw material contained in the aqueous medium, in terms of the concentration of carbohydrates contained therein with respect to the aqueous medium, is usually not less than 5% (w/v), preferably not less than 10% (w/v), and usually not more than 30% (w/v), preferably not more than 20% (w/v). The organic raw material may be further added as the organic raw material decreases due to the progress of the mucic acid-producing reaction.

The pH of the aqueous medium during the mucic acid-producing reaction is preferably adjusted within a range that most effectively allows production of the activity depending on the type of the microorganism of the present invention used. More specifically, in cases where coliform bacteria is used, the pH of the reaction liquid is usually not less than 4.5, preferably not less than 5.0, more preferably not less than 5.5, still more preferably not less than 6.0, and usually not more than 9, preferably not more than 8.5, more preferably not more than 8.0. In cases where a coryneform bacterium is used, the pH of the reaction liquid is usually not less than 5.0, preferably not less than 5.5, more preferably not less than 6.0, still more preferably not less than 6.5, and usually not more than 10, preferably not more than 9.5, more preferably not more than 9.0. In cases where a yeast is used, the pH of the reaction liquid is usually not less than 2.5, preferably not less than 3.0, more preferably not less than 3.5, still more preferably not less than 4.0, and usually not more than 8.5, preferably not more than 8.0, more preferably not more than 7.5.

The pH of the aqueous medium can be adjusted by addition of sodium carbonate, sodium bicarbonate, potassium carbonate, potassium bicarbonate, magnesium carbonate, ammonium carbonate, ammonium bicarbonate, sodium hydroxide, calcium hydroxide, magnesium hydroxide, ammonia (ammonium hydroxide), a mixture thereof, or the like. In cases where the by-product produced in the mucic acid-producing reaction is a basic substance, the pH can be adjusted by adding an inorganic acid such as hydrochloric acid, sulfuric acid, or phosphoric acid; an organic acid such as acetic acid; a mixture thereof; or the like; or by supplying carbon dioxide gas.

The amount of the microbial cells of the microorganism to be used for the mucic acid-producing reaction is not limited. The amount, in terms of the wet microbial cell weight, is usually not less than 1 g/L, preferably not less than 10 g/L, more preferably not less than 20 g/L, and usually not more than 700 g/L, preferably not more than 500 g/L, more preferably not more than 400 g/L.

The period of the mucic acid-producing reaction is not limited. The period is usually not less than 1 hour, preferably not less than 3 hours, and usually not more than 168 hours, preferably not more than 72 hours.

Regarding the temperature during the mucic acid-producing reaction, the reaction may be carried out at the same temperature as the optimum growth temperature of the microorganism used. It is, however, advantageous to carry out the reaction at a temperature higher than the optimum growth temperature. The reaction is preferably carried out at a temperature which is usually 2°C to 20°C higher, preferably 7°C to 15°C higher than the optimum growth temperature. More specifically, in cases of coliform bacteria, the temperature is usually not less than 30°C, preferably not less than 35°C, more preferably not less than 37°C, and usually not more than 45°C, preferably not more than 43°C, more preferably not more than 40°C. In cases of coryneform bacteria, the temperature is usually not less than 30°C, preferably not less than 35°C, more preferably not less than 39°C, and usually not more than 45°C, preferably not more than 43°C, more preferably not more than 41°C. In cases of yeasts, the temperature is usually not less than 27°C, preferably not less than 30°C, more preferably not less than 35°C, and usually not more than 45°C, preferably not more than 43°C, more preferably not more than 40°C.

During the mucic acid-producing reaction, the temperature does not need to be always within the range of 27°C to 45°C. The temperature is preferably within the above-described range for not less than 50%, more preferably not less than 80% of the total reaction time.

Although the mucic acid-producing reaction may be carried out in an anaerobic atmosphere with neither aeration nor supply of oxygen, the reaction is preferably carried out under aeration with stirring. More specifically, the oxygen transfer rate is usually not less than 0 mmol/L/h, preferably not less than 10 mmol/L/h, more preferably not less than 20 mmol/L/h, and usually not more than 200 mmol/L/h, preferably not more than 150 mmol/L/h, more preferably not more than 100 mmol/L/h.

The mode of the method for producing mucic acid in the second method for producing mucic acid of the present invention is not limited, and the method may be applied to any of a batch reaction, semi-batch reaction, and continuous reaction.

### (Recovery Step)

In the second method for producing mucic acid of the present invention, the mucic acid produced by the above-described mucic acid-producing reaction can be accumulated in the reaction liquid. A step of recovering the accumulated mucic acid from the aqueous medium according to a conventional method may be further included. More specifically, for example, by removing solids such as microbial cells by centrifugation, filtration, or the like, and then performing recrystallization or acid precipitation, highly pure mucic acid can be recovered.

For recovery of mucic acid with even higher purity, for example, after conversion into a mucic acid ester, water-soluble impurities may be washed away with water, or, after dissolving the product in an organic solvent and performing separation washing with water, a hydrolysis step may be carried out.

The obtained mucic acid can be used as a raw material of 2,5-furandicarboxylic acid and 2,5-furandicarboxylic acid diester, which are drawing attention as novel polymer raw materials. The mucic acid can also be induced to a variety of other useful chemicals.

### <Method for Producing 2,5-Furandicarboxylic Acid>

After production of mucic acid by the above-described method, the obtained mucic acid can be used as a raw material for production of 2,5-furandicarboxylic acid according to a conventional method. Specific examples of the method for production of 2,5-furandicarboxylic acid include a method in which dehydrocyclization is carried out in the presence of an acid catalyst, and the later-described method in which 2,5-furandicarboxylic acid diester is hydrolyzed.

The acid catalyst used is not limited as long as the reaction can be allowed to proceed. Examples of the acid catalyst include sulfonic acid compounds such as para-toluenesulfonic acid, benzenesulfonic acid, methanesulfonic acid, and camphorsulfonic acid; inorganic acids such as sulfuric acid, phosphoric acid, hydrobromic acid, and hydrochloric acid; and heteropolyacids such as phosphotungstic acid and silicotungstic acid. From the viewpoint of reactivity, para-toluenesulfonic acid or hydrobromic acid is preferred.

The reaction temperature is preferably not less than 100°C, more preferably not less than 120°C.

The production of furandicarboxylic acid by hydrolysis from furandicarboxylic acid diester can be carried out using a known technique.

2,5-Furandicarboxylic acid can be used as a raw material of polyesters and polyamides, and moreover, by reduction of the carboxylic acid, conversion to diol, which can be used as a raw material of polycarbonates and polyesters, is possible. Thus, since there is a possibility that the existing petroleum-derived synthetic resins can be replaced with biomass-derived products, production techniques for 2,5-furandicarboxylic acid are drawing attention. Furthermore, the polymers produced using 2,5-furandicarboxylic acid characteristically have excellent gas barrier properties.

### <Method for Producing 2,5-Furandicarboxylic Acid Diester>

After production of mucic acid by the above-described method, the obtained mucic acid can be used as a raw material for production of 2,5-furandicarboxylic acid diester according to a conventional method. Specific examples of the method for production of 2,5-furandicarboxylic acid diester include a method in which dehydrocyclization is carried out in the presence of a solvent and an acid catalyst, a method in which dehydration is carried out with 2,5-furandicarboxylic acid and alcohol, and a method in which conversion to the corresponding acid chloride is followed by reaction with alcohol.

The acid catalyst for the dehydrocyclization in the presence of the solvent and the acid catalyst is not limited as long as this reaction can be allowed to proceed. Examples of the acid catalyst include sulfonic acid compounds such as para-toluenesulfonic acid, benzenesulfonic acid, methanesulfonic acid, and camphorsulfonic acid; inorganic acids such as sulfuric acid, phosphoric acid, hydrobromic acid, and hydrochloric acid; and heteropolyacids such as phosphotungstic acid and silicotungstic acid. From the viewpoint of reactivity, para-toluenesulfonic acid, phosphoric acid, or sulfuric acid is preferred.

For direct production of the ester, the reaction is preferably carried out while separating water. As the solvent capable of oil-water separation, an alcohol having not less than four carbon atoms is preferred. From the viewpoint of treatment after the reaction, an alcohol having not more than eight carbon atoms is preferred. Although use of a single solvent is preferred from the viewpoint of recovery of the solvent, two or more solvents may be used in combination at arbitrary ratios.

In cases where the conversion to the ester is carried out after the synthesis of 2,5-furandicarboxylic acid, methyl ester or ethyl ester is preferred from the viewpoint of reactivity in the polymerization. For the production of furandicarboxylic acid diester by the method in which dehydration is carried out with 2,5-furandicarboxylic acid and alcohol, or the method in which conversion to the corresponding acid chloride is followed by reaction with alcohol, known techniques may be used

2,5-Furandicarboxylic acid diester can be used as a raw material of polyesters and polyamides, and moreover, by reduction of the ester moiety, conversion to diol, which can be used as a raw material of polycarbonates and polyesters, is possible. Thus, since there is a possibility that the existing petroleum-derived synthetic resins can be replaced with biomass-derived products, production techniques for 2,5-furandicarboxylic acid diester are drawing attention.

Furthermore, the polymers produced using 2,5-furandicarboxylic acid diester characteristically have excellent gas barrier properties.

### EXAMPLES

The present invention is described below in more detail by way of Examples. However, the present invention is not limited by these Examples.

### [Example 1]

For the *E. coli* BW25113 strain, the one-step inactivation method (Proc. Natl. Acad. Sci., 2000, Vol. 97(12), p 6640-5) was carried out as follows to destroy the *galE* gene, the *galT* gene, the *uxaC* gene, the *garD* gene, the *pgi* gene, the arnA gene, and the *agp* gene to prepare the *E. coli* BW25113/*ΔgalET*/*ΔuxaC*/*ΔgarD*/*Δpgi*/*ΔarnA*/*Δagp* strain.

A kanamycin resistance gene having, at each of both ends, a sequence having homology to the flanking region of the *galET* gene, and FRT (FLP Recognition Target), which is a recognition site of FLP recombinase, was amplified by PCR using pKD13 as a template, and the gETF and gETR primers (SEQ ID NOs:1 and 2). The resulting PCR product was used for transformation of the BW25113 strain having pKD46, which is a plasmid capable of induced expression of Red recombinase with arabinose. Transformants that could be confirmed, by colony PCR, to have undergone replacement of the *galET* gene with the kanamycin resistance gene were subjected to curing of pKD46, followed by introduction of pCP20, which is a helper plasmid capable of expressing the FLP enzyme. A strain from which the kanamycin resistance gene was eliminated as a result of the recombination by the FLP enzyme, and for which curing of pCP20 could be confirmed, was provided as BW25113/*ΔgalET*.

In the same manner as described above, using the BW25113 strain having pKD46, a strain in which the *uxaC* gene was replaced with the kanamycin resistance gene was prepared, followed by performing P1 transduction into BW25113/*ΔgalET*. To the resulting strain, the helper plasmid pCP20 was introduced to eliminate the kanamycin resistance gene. By this, a strain in which the *galET* gene and the *uxaC* gene were deleted was constructed to provide BW25113/*ΔgalET*/*ΔuxaC.*

By repeating the above method, a strain in which deletion of the *garD* gene was combined was constructed to provide BW25113/*ΔgalET*/*ΔuxaC*/*ΔgarD.*

### [Example 2]

By the same method as in Example 1, the *galE* gene, the *galT* gene, the *uxaC* gene, the *garD* gene, the *pgi* gene, the arnA gene, and the *agp* gene were destroyed to prepare the *E. coli* BW25113/*ΔgalET*/*ΔuxaC*/*ΔgarD*/*Δpgi* strain.

### [Example 3]

By the same method as in Example 1, the *galE* gene, the *galT* gene, the *uxaC* gene, the *garD* gene, the *pgi* gene, the *arnA* gene, and the *agp* gene were destroyed to prepare the *E. coli* BW25113/*ΔgalET*/*ΔuxaC*/*ΔgarD*/*Δpgi*/*ΔarnA*/*Δagp* strain.

### [Example 4]

In the BW25113/*ΔgalET*/*ΔuxaC*/*ΔgarD* strain prepared in Example 1, the *ugd* gene, the *galU* gene, the *galF* gene, the *pgm* gene, the *udh* gene, the *agp* gene, the *LmjF17.1160* gene, and the *cap1J* gene were enhanced as described below to prepare the *E. coli* BW25113/pZA_P_{LlacO1}*_ugd_galU_galF_pgm*/pZE_P_{LlacO1}*_udh_agp_Lmj F17.1160_cap1J*/*ΔgalET*/*ΔuxaC*/*ΔgarD* strain.

The *ugd* gene, the *galU* gene, the *galF* gene, and the *pgm* gene were amplified by PCR using the *E. coli* genome as a template, and ugdF/ugdR (SEQ ID NOs: 3 and 4), galUF/galUR (SEQ ID NOs: 5 and 6), galFF/galFR (SEQ ID NOs: 7 and 8), and pgmF/pgmR (SEQ ID NOs: 9 and 10), respectively, as primer combinations. The plasmid backbone of the *E. coli* expression vector pZA (ChemSusChem, 2011, Vol. 4(8), p 1068-70) was amplified by PCR using the pZAR and pZAF primers (SEQ ID NOs:11 and 12). By the Gibson Assembly method (Nat. Methods, 2009, Vol.6(5), p 343-5), the *ugd* gene, the *galU* gene, the *galF* gene, and the *pgm* gene were inserted downstream of the P_{LlacO1} promoter of the pZA vector. The constructed plasmid was designated pZA_P_{LlacO1}*_ugd_galU_galF_pgm.*

The *agp* gene derived from *Escherichia coli* was amplified by PCR using the *E. coli* genome as a template, and the agpF and agpR primers (SEQ ID NOs:13 and 14). For improving expression, in *E. coli,* of each of the *udh* gene derived from *Pseudomonas putida,* the *LmjF17.1160* gene derived from *Leishmania major,* and the *cap1J* gene derived from *Streptococcus pneumoniae,* their sequences were synthesized with optimized codons (SEQ ID NOs:15, 16, and 17, respectively). Amplification by PCR was carried out using these synthetic genes as templates, and udhF/udhR (SEQ ID NOs:18 and 19), LmjF17.1160F/LmjF17.1160R (SEQ ID NOs:20 and 21), and cap1JF/cap1JR (SEQ ID NOs:22 and 23), respectively, as primer combinations. The plasmid backbone of the *E. coli* expression vector pZE (ChemSusChem, 2011, Vol. 4(8), p 1068-70) was prepared by treatment with the restriction enzymes *Acc*65I and *Xba*I, and the *udh* gene, the *agp* gene, the *LmjF17.1160* gene, and the *cap1J* gene were inserted downstream of the P_{LlacO1} promoter in the pZE vector by the GibsonAssembly method (Nat. Methods, 2009, Vol. 6(5), p 343-5). The constructed plasmid was designated pZE_P_{LlacO1}*_udh_agp_LinjF17.1160_cap1J.*

The BW25113/*ΔgalET*/*ΔuxaC*/*ΔgarD* strain prepared in Example 1 was transformed with the plasmids pZA_P_{LlacO1}*_ugd_galU_galF_pgm* and pZE_P_{LlacO1}_*udh_agp_LmjF17.1160_cap1J* constructed as described above, and the obtained strain was provided as BW25113/pZA_P_{LlacO1}*_ugd_galU_galF_pgm*/pZE_P_{LlacO1}*_udh_agp_Lmj F17.1160_cap1J*/*ΔgalET*/*ΔuxaC*/*ΔgarD.*

### [Example 5]

In the BW25113/*ΔgalET*/*ΔuxaC*/*ΔgarD* strain prepared in Example 1, the *ugd* gene, the *galU* gene, the *galF* gene, the *pgm* gene, the *udh* gene, the mutant *agp* gene (R94H), the *LmjF17.1160* gene, and the *cap1J* gene were enhanced as described below to prepare the *E. coli* BW25113/pZA_P_{LlacO1}*_ugd_galU_galf_pgm*/pZE-P_{LlacO1}*_udh_agp94H_ LmjF17.1160_cap1J*/*ΔgalET*/*ΔuxaC*/*ΔgarD* strain.

The *agp* gene derived from *Escherichia coli* in which the arginine at position 94 was mutated to histidine was amplified by overlap PCR (Methods. Mol. Biol., 2003, Vol. 226, p 511-6) using the *E. coli* genome as a template, and agpF and agpR94HR (SEQ ID NOs:13 and 24), and agpR94HF and agpR (SEQ ID NOs:25 and 14) as primer combinations. In the same manner as in the case of pZE_P_{LlacO1}*_udh_agp_LmjF17.1160_cap1J* in Example 4 except for the above process, a plasmid was constructed, and designated pZE_P_{LlacO1}*_udh_agp94H_LmjF17.1160_cap1J.*

The BW25113/*ΔgalET*/*ΔuxaC*/*ΔgarD* strain prepared in Example 1 was transformed with the plasmids pZA_P_{LlacO1}*_ugd_galU_galF_pgm* and pZE_P_{LlacO1}*_udh_agp94H_LmjF17.1160_cap1J* constructed as described above, and the obtained strain was provided as BW25113/pZA_P_{LlacO1}_*ugd_galU_galf_pgm*/pZE-P_{LlacO1}*_udh_agp94H_ LmjF17.1160_cap1J*/*ΔgalET*/*ΔuxaC*/*ΔgarD.*

### [Example 6]

In the BW25113/*ΔgalET*/*ΔuxaC*/*ΔgarD* strain prepared in Example 1, the *ugd* gene, the *galU* gene, the *galF* gene, the *pgm* gene, the *udh* gene, the mutant *agp* gene (M123V), the *LmjF17.1160* gene, and the *cap1J* gene were enhanced as described below to prepare the *E. coli* BW25113/pZA_P_{LlacO1}*_ugd_galU_galF_pgl*/pZE_P_{LlacO1}*_udh_agp123V _LmjF17.1160_cap1J*/*ΔgalET*/*ΔuxaC*/*ΔgarD* strain.

The *agp* gene derived from *Escherichia coli* in which the methionine at position 123 was mutated to valine was amplified by overlap PCR (Methods. Mol. Biol., 2003, Vol. 226, p 511-6) using the *E. coli* genome as a template, and agpF and agpM123VR (SEQ ID NOs:13 and 26), and agpM123VF and agpR (SEQ ID NOs:27 and 14) as primer combinations. In the same manner as in the case of pZE_P_{LlacO1}*_udh_agp_LmjF17.1160_cap1J* in Example 4 except for the above process, a plasmid was constructed, and designated pZE_P_{LlacO1}*_udh_agp123V_LmjF17.1160_cap1J.*

The BW25113/*ΔgalET*/*ΔuxaC*/*ΔgarD* strain prepared in Example 1 was transformed with the plasmids pZA_P_{LlacO1}*_ugd_galU_galF_pgm* and pZE_P_{LlacO1}*_udh_agp123V_LmjF17.1160_cap1J* constructed as described above, and the obtained strain was provided as BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD.*

### [Example 7]

In the BW25113/*ΔgalET*/*ΔuxaC*/*ΔgarD* strain prepared in Example 1, the *ugd* gene, the *galU* gene, the *galF* gene, the *pgm* gene, the *udh* gene, the mutant *agp* gene (M123A), the *LmjF17.1160* gene, and the *cap1J* gene were enhanced as described below to prepare the *E. coli* BW25113/pZA_P_{LlacO1}*_ugd_galU_galF_pgm*/pZE_P_{LlacO1}*_udh_agp123A _LmjF17.1160_cap1J*/*ΔgalET*/*ΔuxaC*/*ΔgarD* strain.

The *agp* gene derived from *Escherichia coli* in which the methionine at position 123 was mutated to alanine was amplified by overlap PCR (Methods. Mol. Biol., 2003, Vol. 226, p 511-6) using the *E. coli* genome as a template, and agpF and agpM123AR (SEQ ID NOs:13 and 28), and agpM123AF and agpR (SEQ ID NOs:29 and 14) as primer combinations. In the same manner as in the case of pZE_P_{LlacO1}*_udh_agp_LmjF17.1160_cap1J* in Example 4 except for the above process, a plasmid was constructed, and designated pZE_P_{LlacO1}*_udh_agp123A_LmjF17.1160_cap1J.*

The BW25113/*ΔgalET*/*ΔuxaC*/*ΔgarD* strain prepared in Example 1 was transformed with the plasmids pZA_P_{LlacO1}*_ugd_galU_galF_pgm* and pZE_P_{LlacO1}*_udh_agp123A_LmjF17.1160_cap1J* constructed as described above, and the obtained strain was provided as BW25113/pZA-P_{LlacO1}*_ugd_galU_galF_pgm*/pZE_P_{LlacO1}*_udh_agp123A _LmjF17.1160_cap1J*/*ΔgalET*/*ΔuxaC*/*ΔgarD.*

### [Example 8]

In the BW25113/*ΔgalET*/*ΔuxaC*/*ΔgarD* strain prepared in Example 1, the *ugd* gene, the *galU* gene, the *galF* gene, the *pgm* gene, the *udh* gene, the mutant *agp* gene (E196D), the *LmjF17.1160* gene, and the *cap1J* gene were enhanced as described below to prepare the *E. coli* BW25113/pZA_P_{LlacO1}_*ugd_galU_galF_pgm*/pZE_P_{LlacO1}_*udh_agp196D _LmjF17.1160_cap1J*/*ΔgalET*/*ΔuxaC*/*ΔgarD* strain.

The *agp* gene derived from *Escherichia coli* in which the glutamic acid at position 196 was mutated to aspartic acid was amplified by overlap PCR (Methods. Mol. Biol., 2003, Vol. 226, p 511-6) using the *E. coli* genome as a template, and agpF and agpE196DR (SEQ ID NOs:13 and 30), and agpE196DF and agpR (SEQ ID NOs:31 and 14) as primer combinations. In the same manner as in the case of pZE_P_{LlacO1}*_udh_agp_LmjF17.1160_cap1J* in Example 4 except for the above process, a plasmid was constructed, and designated pZE_P_{LlacO1}*_udh_agp196D_LmjF17.1160_cap1J.*

The BW25113/*ΔgalET*/*ΔuxaC*/*ΔgarD* strain prepared in Example 1 was transformed with the plasmids pZA_P_{LlacO1}*_ugd_galU_galF_pgm* and pZE_P_{LlacO1}*_udh_agp196D_LmjF17.1160_cap1J* constructed as described above, and the obtained strain was provided as BWS25113/pZA_P_{LlacO1}*_ugd_galU_galF_pgm*/pZE_P_{LlacO1}*_udh_agp196D _LmjF17.1160_cap1J*/*ΔgalET*/*ΔuxaC*/*ΔgarD.*

### [Example 9]

In the BW25113/*ΔgalET*/*ΔuxaC*/*ΔgarD*/*Δpgi* strain prepared in Example 2, the *ugd* gene, the *galU* gene, the *galF* gene, the *pgm* gene, the *udh* gene, the mutant *agp* gene (M123V), the *LmjF17.1160* gene, and the *cap1J* gene were enhanced as described below to prepare the *E. coli* BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi* strain.

The BW25113/*ΔgalET*/*ΔuxaC*/*ΔgarD*/*Δpgi* strain prepared in Example 2 was transformed with the plasmids pZA_P_{LlacO1}*_ugd_galU_galF_pgm* and pZE_P_{LlacO1}*_udh_agp123V_LmjF17.1160_cap1J* constructed in Examples 4 and 6, and the obtained strain was provided as BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi.*

### [Example 10]

In the BW25113/*ΔgalET*/*ΔuxaC*/*ΔgarD*/*Δpgi*/*ΔarnA*/*Δagp* strain prepared in Example 3, the *ugd* gene, the *galU* gene, the *galF* gene, the *pgm* gene, the *udh* gene, the mutant *agp* gene (M123V), the *LmjF17.1160* gene, and the *cap1J* gene were enhanced as described below to prepare the *E. coli* BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi*/*ΔarnA*/*Δagp* strain.

The BW25113/*ΔgalET*/*ΔuxaC*/*ΔgarD*/*Δpgi*/*ΔarnA*/*Δagp* strain prepared in Example 3 was transformed with the plasmids pZA_P_{LlacO1}*_ugd_galU_galF_pgm* and pZE_P_{LlacO1}*_udh_agp123V_LmjF17.1160_cap1J* constructed in Examples 4 and 6, and the obtained strain was provided as BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi*/*ΔarnA*/*Δagp.*

### [Example 11]

Using the BW25113/pZA_P_{LlacO1}*_ugd_galU_galF_pgm*/pZE_P_{LlacO1}*_udh_agp_Lmj F17.1160_cap1J*/*ΔgalET*/*ΔuxaC*/*ΔgarD* strain prepared in Example 4, evaluation of mucic acid fermentation by flask culture was carried out as follows.

The BW25113/pZA_P_{LlacO1}*_ugd_galU_galF_pgm*/pZE_P_{LlacO1}*_udh_agp_Lmj F17.1160_cap1J*/*ΔgalET*/*ΔuxaC*/*ΔgarD* strain prepared in Example 4 was cultured overnight in 2XYT medium (16 g/L Bacto-trypton, 10 g/L Yeast extract, 5 g/L NaCl). In a 125-mL flask, 5 mL of M9 medium containing 4% glucose, 5 g/L Yeast extract, 0.5 mM IPTG, 50 µg/mL kanamycin, and 50 µg/mL ampicillin was placed, and the above culture liquid was inoculated such that 1/25 dilution was achieved. Culture was then performed at 30°C for 48 hours. The culture liquid was analyzed by HPLC in three replicates under the same conditions, and the average was calculated. As a result, 15.3 mg/L of mucic acid was detected.

### [Example 12]

Using the BW25113/pZA_P_{LlacO1}*_ugd_galU_galF_pgm*/pZE_P_{LlacO1}*_udh_agp94H_ LmjF17.1160_cap1J*/*ΔgalET*/*ΔuxaC*/*ΔgarD* strain prepared in Example 5, evaluation of mucic acid fermentation by flask culture was carried out in the same manner as in Example 11. As a result, 14.0 mg/L of mucic acid was detected.

### [Example 13]

Using the BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD* strain prepared in Example 6, evaluation of mucic acid fermentation by flask culture was carried out in the same manner as in Example 11. As a result, 19.8 mg/L of mucic acid was detected.

### [Example 14]

Using the BW25113/pZA_P_{LlacO1}*_ugd_galU_galF_pgm*/pZE_P_{LlacO1}*_udh_agp123A _LmjF17.1160_cap1J*/*ΔgalET*/*ΔuxaC*/*ΔgarD* strain prepared in Example 7, evaluation of mucic acid fermentation by flask culture was carried out in the same manner as in Example 11. As a result, 17.7 mg/L of mucic acid was detected.

### [Example 15]

Using the BW25113/pZA_P_{LlacO1}*_ugd_galU_galF_pgm*/pZE_P_{LlacO1}*_udh_agp196D _LmjF17.1160_cap1J*/*ΔgalET*/*ΔuxaC*/*ΔgarD* strain prepared in Example 8, evaluation of mucic acid fermentation by flask culture was carried out in the same manner as in Example 11. As a result, 18.9 mg/L of mucic acid was detected.

### [Example 16]

Using the BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi* strain prepared in Example 9, evaluation of mucic acid fermentation by flask culture was carried out as follows.

The evaluation was carried out in the same manner as in Example 11 except that, for the culture using a 125-mL flask, M9 medium containing 2% glucose, 2% glycerol, 5 g/L Yeast extract, 0.5 mM IPTG, 50 µg/mL kanamycin, and 50 µg/mL ampicillin was used. As a result, 130 mg/L of mucic acid was detected.

### [Example 17]

Using the BW25113/pZA_P_{LlacO1}*_ugd_galU_galF_pgm*/pZE_P_{LlacO1}*_udh_agp123V _LmjF17.1160_cap1J*/*ΔgalET*/*ΔuxaC*/*ΔgarD*/*Δpgi*/*ΔarnA*/*Δagp* strain prepared in Example 10, evaluation of mucic acid fermentation by flask culture was carried out in the same manner as in Example 16. As a result, 214 mg/L of mucic acid was detected.

### INDUSTRIAL APPLICABILITY

By the present invention, a method and a microorganism that allow efficient production of mucic acid from a common organic raw material such as glucose or galactose can be provided.

By using such a microorganism of the present invention, mucic acid can be efficiently produced. Furthermore, 2,5-furandicarboxylic acid and 2,5-furandicarboxylic acid diester can be efficiently produced using the mucic acid obtained by the production method of the present invention as a raw material. Thus, the present invention is highly industrially applicable.

## Claims

1. A method for producing mucic acid, comprising:
using glucose or galactose as a starting material or an intermediate; and
obtaining mucic acid via UDP-galacturonic acid and galacturonic acid, which are produced sequentially as intermediates.

2. The method for producing mucic acid according to claim 1, wherein
glucose is used as a starting material or an intermediate, and
mucic acid is obtained via UDP-glucose, UDP-galacturonic acid, and galacturonic acid, which are produced sequentially as intermediates.

3. The method for producing mucic acid according to claim 2, wherein mucic acid is obtained via UDP-glucuronic acid, which is produced as an intermediate after UDP-glucose and before UDP-galacturonic acid.

4. The method for producing mucic acid according to claim 2, wherein mucic acid is obtained via UDP-galactose, which is produced as an intermediate after UDP-glucose and before UDP-galacturonic acid.

5. The method for producing mucic acid according to claim 1, wherein galactose is used as a starting material or an intermediate, and mucic acid is obtained via UDP-galactose, UDP-galacturonic acid, and galacturonic acid, which are produced sequentially as intermediates.

6. A microorganism modified to have enhanced UDP-galacturonate pyrophosphorylase activity and to have at least one enhanced enzyme activity selected from the group consisting of UDP-glucuronate 4-epimerase activity, UDP-galactose 6-dehydrogenase activity, and galacturonate dehydrogenase activity, as compared to non-modified strains.

7. The microorganism according to claim 6, wherein
the microorganism is modified to have enhanced UDP-glucuronate 4-epimerase activity, UDP-galacturonate pyrophosphorylase activity, and galacturonate dehydrogenase activity, as compared to non-modified strains.

8. The microorganism according to claim 7, wherein
the microorganism is modified to further have at least one enhanced enzyme activity selected from the group consisting of glucokinase activity, phosphoglucomutase activity, glucose-1-phosphate uridylyltransferase activity, UDP-glucose 6-dehydrogenase activity, and galacturonate-1-phosphate phosphatase activity, as compared to non-modified strains.

9. The microorganism according to claim 7 or 8, wherein
the microorganism is modified to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, hexose-1-phosphate uridylyltransferase activity, UDP-glucose 4-epimerase activity, UDP-glucuronic acid dehydrogenase activity, galacturonate isomerase activity, galacturonate reductase activity, and galactarate dehydratase activity, as compared to non-modified strains.

10. The microorganism according to claim 6, wherein
the microorganism is modified to have enhanced UDP-galactose 6-dehydrogenase activity, UDP-galacturonate pyrophosphorylase activity, and galacturonate dehydrogenase activity, as compared to non-modified strains.

11. The microorganism according to claim 10, wherein
the microorganism is modified to further have at least one enhanced enzyme activity selected from the group consisting of glucokinase activity, phosphoglucomutase activity, glucose-1-phosphate uridylyltransferase activity, hexose-1-phosphate uridylyltransferase activity, UDP-glucose 4-epimerase activity, galactokinase activity, UDP-galactose pyrophosphorylase activity, and galacturonate-1-phosphate phosphatase activity, as compared to non-modified strains.

12. The microorganism according to claim 10 or 11, wherein
the microorganism is modified to further have at least one decreased enzyme activity selected from the group consisting of glucose-6-phosphate 1-dehydrogenase activity, glucose-6-phosphate isomerase activity, glucose-1-phosphate phosphatase activity, ADP-glucose pyrophosphorylase activity, UDP-glucose 6-dehydrogenase activity, UDP-glucuronic acid dehydrogenase activity, galacturonate isomerase activity, galacturonate reductase activity, and galactarate dehydratase activity, as compared to non-modified strains.

13. The microorganism according to any one of claims 6 to 12, wherein the microorganism is at least one selected from the group consisting of coliform bacteria, coryneform bacteria, bacteria belonging to the genus *Bacillus,* bacteria belonging to the genus *Lactobacillus,* bacteria belonging to the genus *Actinobacillus,* bacteria belonging to the genus *Pseudomonas,* filamentous fungi, and yeasts.

14. A method for producing mucic acid, comprising:
allowing the microorganism according to any one of claims 6 to 13 or a treated product of the microorganism to act on an organic raw material in an aqueous medium.

15. The method for producing mucic acid according to claim 14, wherein the organic raw material contains at least one selected from the group consisting of glucose, sucrose, galactose, and lactose.

16. A method for producing 2,5-furandicarboxylic acid, comprising the steps of:
(i) producing mucic acid by the method according to any one of claims 1 to 5, 14, and 15; and
(ii) converting the mucic acid obtained in the step (i) into 2,5-furandicarboxylic acid.

17. A method for producing 2,5-furandicarboxylic acid diester, comprising the steps of:
(i) producing mucic acid by the method according to any one of claims 1 to 5, 14, and 15; and
(ii) converting the mucic acid obtained in the step (i) into 2,5-furandicarboxylic acid diester.
